# EUROPEAN PATENT APPLICATION

(11) **EP 4 206 188 A1**
(43) Date of publication of application: **05.07.2023**
(21) Application number: 21860463.5
(22) Date of filing: 26.08.2021
(51) Int. Cl.: C07D 209/10, C09K 11/06, A61K 49/00

(54) **TUMOR CONTRAST COMPOUND, PREPARATION METHOD THEREFOR AND APPLICATION THEREOF IN TUMOR DIAGNOSTIC IMAGING**

(30) Priority: 28.08.2020 CN 202010885318
(71) Applicant: Ningbo Haibo Biotechnology Co., LTD, Ningbo, Zhejiang 315615 (CN)
(72) Inventor: WANG, Weiwei, Shanghai 201318 (CN)
(74) Representative: V.O.
(86) International application number: PCT/CN2021/114672
(87) International publication number: WO 2022/042628

(57) **Abstract**

Disclosed are a tumor contrast compound, a preparation method therefor and an application thereof in tumor diagnostic imaging. A contrast agent of the present invention comprises the compound as represented by formula I in this text and anionic surfactant. Near-infrared real-time imaging having high contrast ratio and clear and distinguishable imaging results can be achieved by using the contrast agent of the present invention.

## Description

### Technical field

The present disclosure relates to a tumor contrast compound, a preparation method thereof and their application in tumor diagnostic imaging.

### Background

Molecular imaging is an interdiscipline formed by the integration of molecular biology and medical imaging. Compared with traditional imaging examinations, molecular imaging technology has the advantages of high sensitivity and quantitativeness. Molecular imaging technology or molecular displaying technology mainly includes magnetic resonance (MR) molecular imaging, nuclear medicine molecular imaging and optical molecular imaging. Among them, near-infrared fluorescence (NIRF) imaging technology in optical molecular imaging technology has been widely used in clinical surgery, and the basic principle is as follows: near-infrared light emitted by fluorescent tracers after excitation is utilized to identify blood vessels, lymph nodes and tumor lesions and the like, and assist clinicians in surgical operations. At present, indocyanine green (ICG) has been widely used in clinical fields as a near-infrared fluorescent tracer. However, because ICG does not specifically identify tumor tissue, the use of ICG in surgical tumor resection surgery can only indirectly label tumor lesions through vascular and tissue differences. Its imaging results are poor, which are manifested as insufficient sensitivity, poor tumor specificity, and short imaging time.

### Summary

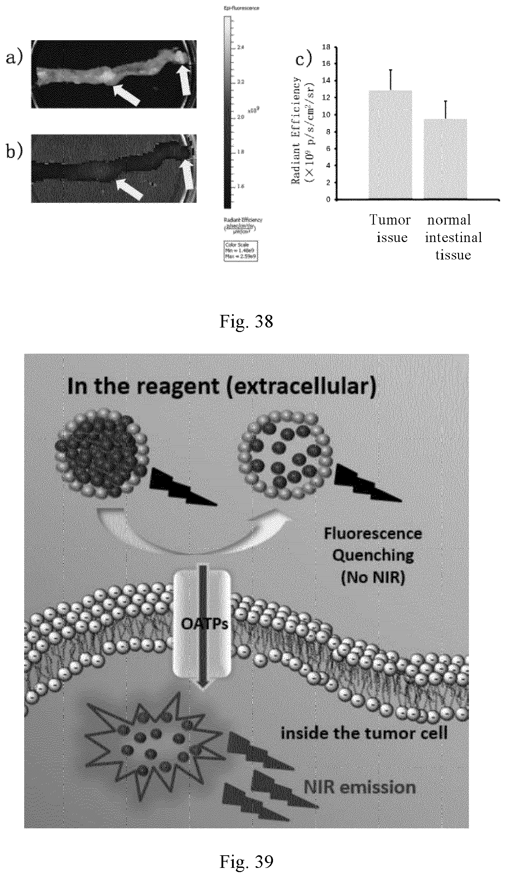

The present disclosure provides a completely new near-infrared fluorescent tumor labeling technology. It is found in the present disclosure that, as shown in Figure 39, anionic surfactants and fluorescent small molecules are dissolved in water to form a stable imaging reagent (also referred to herein as a contrast agent). Anionic surfactants are bonded to fluorescent small molecules in solution, and their binding effect can produce a strong fluorescence quenching effect, resulting in fluorescent small molecules that cannot be excited, and the reagent itself has no fluorescent signal. When the imaging reagent comes into contact with tumor cells, fluorescent small molecules can identify organic anion transporting polypeptides (OATPs) on the surface of tumor cells and quickly enter the tumor cells, while anionic surfactants cannot selectively enter tumor cells, so the fluorescence quenching effect disappears. As a result, fluorescent small molecules are excited inside tumor cells and emit near-infrared fluorescent signals, thereby labeling tumor lesions. Based on the above findings, the present disclosure can provide near infrared real-time imaging with high contrast and clearly identifiable imaging results.

Thus, a first aspect of the present disclosure provides a contrast agent comprising a compound represented by the following formula I and anionic surfactant: 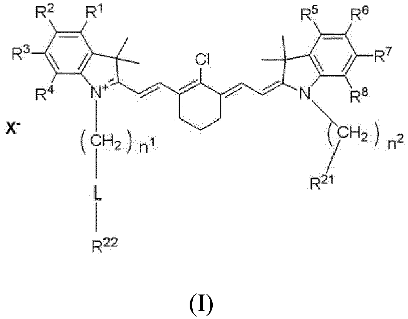 wherein,
X⁻ is anionic ion;
R¹-R⁸ are each independently H, hydroxyl, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, an optionally substituted C₃₋₁₀ cycloalkyl group, an optionally substituted 6-14 membered aryl group, an optionally substituted 5-14 membered heteroaryl group, and an optionally substituted 5-14 membered heterocyclic group; or R¹ and R², R² and R³ or R³ and R⁴ form an optionally substituted 3-8 membered carbon ring, 6-14 membered aromatic ring, 5-14 membered heteroaromatic ring or 5-14 membered hetero-ring with the carbon atoms to which they are attached, respectively, R⁵ and R⁶, R⁶ and R⁷ or R⁷ and R⁸ form an optionally substituted 3-8 membered carbon ring, 6-14 membered aromatic ring, 5-14 membered heteroaromatic ring or 5-14 membered hetero-ring with the carbon atoms to which they are attached, respectively;
R²¹ and R²² are each independently H, halogen, nitro, cyano, -SO₃, -COOH, -SO₃N(Rₐ)₃, C₁₋₆ alkyl, C₂₋₆ alkenyl, C₂₋₆ alkynyl, C₁₋₆ acyl, -NH₂, C₁₋₆ alkoxy, an optionally substituted C₃₋₁₅ cycloalkyl, an optionally substituted C₃₋₁₅ cycloalkyl C₁₋₆ alkyl, an optionally substituted 6-14-membered aryl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl; wherein each Rₐ is independently H and C₁₋₄ alkyl;
n¹ and n² are each independently selected from integers of 0-12, preferably integers of 0-6; and
L is a bond, or a linking group.

In one or more embodiments, L is a bond, n¹ and n² are the same integers, and R²¹ and R²² are the same groups.

In one or more embodiments, the group -(CH₂)n¹-L-R²² is different from the group - (CH₂)n²-R²¹, and the steric hindrance of the group -(CH₂)n¹-L-R²² is greater than that of the group -(CH₂)n²-R²¹.

In one or more embodiments, R¹-R⁸ are all hydrogen.

In one or more embodiments, L is selected from the group consisting of ^{∗}-T₁-C(O)-, *-C(O)-T₁-, ^{∗}-S(O)₂-T₂- and ^{∗}-T₂-S(O)₂-, wherein T₁ is selected from the group consisting of NH, O and S, T₂ is selected from the group consisting of O, NH and S, * indicates the location in connection with -(CH₂)n¹-.

In one or more embodiments, the group -(CH₂)n¹-L-R²² is the same as the group - (CH₂)n²-R²¹, both of which are C₁₋₆ alkyl groups that are optionally substituted by 1 or 2 substituents selected from the group consisting of halogen, nitro, cyano, -SO₃, -COOH and -NH₂. Preferably, the group -(CH₂)n¹-L-R²² is the same as the group -(CH₂)n²-R²¹, and both are C₁₋₆ alkyl groups that are optionally substituted by -COOH.

In one or more embodiments, the group -(CH₂)n¹-L-R²² is different from the group - (CH₂)n²-R²¹, the group -(CH₂)n¹-L-R²² is C₁₋₆ alkyl that is optionally substituted by 1 or 2 substituents selected from the group consisting of halogen, nitro, cyano, -SO₃, -COOH and -NH₂, preferably C₁₋₆ alkyl that is optionally substituted by -COOH; the group -(CH₂)n²-R²¹ is C₁₋₆ alkyl that is optionally substituted by 1 or 2 substituents selected from the group consisting of halogen, nitro, cyano, -SO₃, -COOH and -NH₂, preferably C₁₋₆ alkyl that is optionally substituted by -COOH, and the carbon chain length of the group -(CH₂)n²-R²¹ is shorter than the carbon chain length of the group -(CH₂)n¹-L-R²². Preferably, the group - (CH₂)n¹-L-R²² is C₁₋₆ alkyl that is optionally substituted by -COOH, the group -(CH₂)n²-R²¹ is C₁₋₆ alkyl that is optionally substituted by -COOH, and the carbon chain length of the group -(CH₂)n²-R²¹ is shorter than the carbon chain length of the group -(CH₂)n¹-L-R²². More preferably, the group -(CH₂)n¹-L-R²² is C₁₋₆ alkyl that is substituted by -COOH, the group -(CH₂)n²-R²¹ is an unsubstituted C₁₋₆ alkyl, and the carbon chain length of the group -(CH₂)n²-R²¹ is shorter than the carbon chain length of the group -(CH₂)n¹-L-R²².

In one or more embodiments, the compound of Formula I does not comprise Compound 8.

Preferably, in Formula I, in R¹-R⁸ as well as R²¹ and R²², said an optionally substituted C₃₋₁₀ cycloalkyl, an optionally substituted 6-14-membered aryl, an optionally substituted 5-14-membered heteroaryl group, an optionally substituted 5-14-membered heterocyclic group, an optionally substituted C₃₋₁₅ cycloalkyl, an optionally substituted C₃₋₁₅ cycloalkyl C₁₋₆ alkyl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl are each an optionally substituted by 1-3 substitutents selected from the group consisting of halogen, C₁₋₆ alkyl, halogenated C₁₋₆ alkyl, hydroxyl, carboxyl, amino (including -NH₂ and -NR'R"), C₁₋₆ alkoxy and halogenated C₁₋₆ alkoxy, wherein R' is selected from C₁₋₄ alkyl and C₁₋₆ alkoxycarbonyl, and R" is selected from Hand C₁₋₄ alkyl.

In one or more embodiments, R²² is C₆₋₁₄ aryl that is optionally substituted by 1 or 2 substitutents selected from the group consisting of halogen, C₁₋₆ alkyl, halogenated C₁₋₆ alkyl, amino, C₁₋₆ alkoxy and halogenated C₁₋₆ alkoxy or an optionally substituted C₃₋₁₅ cycloalkyl C₁₋₆ alkyl.

In one or more embodiments, the group -(CH₂)n¹-L-R²² is different from the group - (CH₂)n²-R²¹; wherein R²² is C₆₋₁₄ aryl or C₃₋₁₅ cycloalkyl C₁₋₆ alkyl that is optionally substituted by 1 or 2 substituents selected from the group consisting of halogen, C₁₋₆ alkyl, halogenated C₁₋₆ alkyl, -NH₂, -NR'R", C₁₋₆ alkoxy and halogenated C₁₋₆ alkoxy, wherein R' is selected from C₁₋₄ alkyl and C₁₋₆ alkoxycarbonyl; R²¹ is H, halogen, C₁₋₆ alkyl, halogenated C₁₋₆ alkyl, C₂₋₆ alkenyl or C₂₋₆ alkynyl, and when R²¹ is H, n² is not 0. Preferably, R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl; the group -(CH₂)n²-R²¹ is C₁₋₆ alkyl or halogenated C₁₋₆ alkyl.

In one or more embodiments, R¹-R⁸ are hydrogen; L is ^{∗}-T₁-C(O)- or *- C(O)-T₁-, wherein T₁ is selected from the group consisting of NH, O and S; n¹ and n² are each integer of 1 to 6; the group -(CH₂)n²-R²¹ is C₁₋₆ alkyl; R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from Hand C₁₋₄ alkyl.

In one or more embodiments, the anion is selected from the group consisting of F⁻, Cl⁻, Br⁻, I⁻, NO₃⁻, SO₄²⁻ , PO₄³⁻, HPO₄²⁻, H₂PO₄⁻, CO₃²⁻, HCO₃⁻, SO₃²⁻, HSO₃⁻, CH₃COO⁻ and CH₃SO₃⁻.

In one or more embodiments, the compound of Formula I according to the present disclosure has the structure represented by the following formula Ia, Ib or Ic: 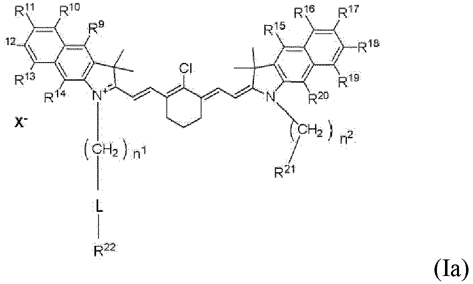 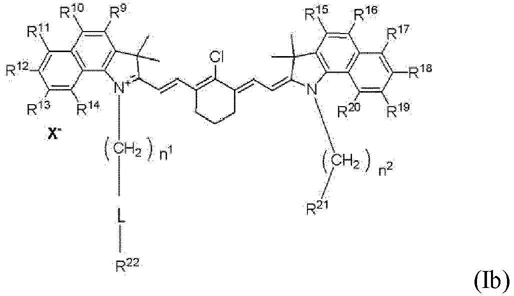 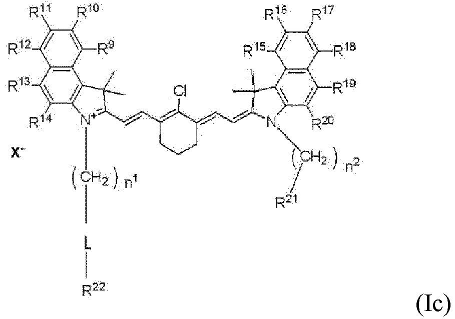 in each formula, n¹, n², L, R²¹ and R²² are as described in any of the foregoing embodiments;
R⁹-R²⁰ are each independently selected from the group consisting of H, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, C₃₋₁₀ cycloalkyl, C₆₋₁₄ aryl, C₅₋₁₄ heteroaryl and C₃₋₁₀ heterocyclic groups. Preferably, R⁹-R²⁰ are each independently H, halogen and C₁₋₆ alkyl, more preferably H.

In one or more embodiments, the compound of Formula I is one or more selected from the following compounds:

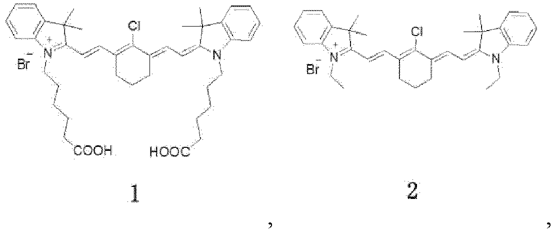

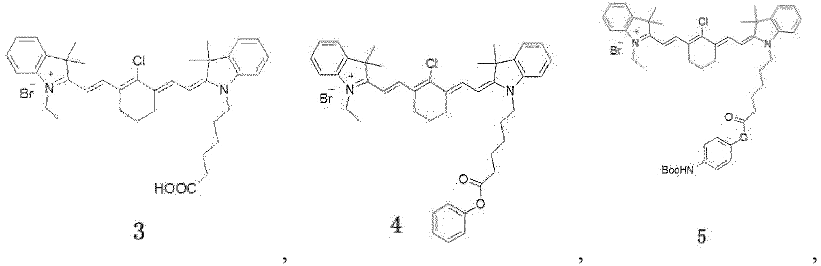

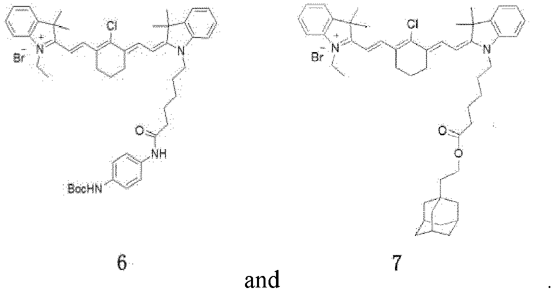

In one or more embodiments, the molar ratio of the compound of Formula I to the anionic surfactant in the contrast agent is 1: (0.1-20), such as 1: (0.1-10) or 1: (0.2-5).

In one or more embodiments, the anionic surfactant is selected from carboxylates, sulfonates, sulfate salts, phosphate ester salts and lipopeptide anionic surfactants; preferably, the anionic surfactant is selected from sulfonates and lipopeptide anionic surfactants; more preferably, the anionic surfactant is selected from the group consisting of surfactin, sodium lauryl sulfonate and sodium lauryl benzene sulfonate.

In one or more embodiments, the final concentration of the compound of Formula I in the contrast agent is in the range of 1 ∼ 100 µM, such as 5 ∼ 50 µM or 10 ∼ 30 µM.

In one or more embodiments, the contrast agent is a lyophilized powder.

In one or more embodiments, the contrast agent is in the form of an aqueous solution.

A second aspect of the present disclosure provides a compound represented by the following Formula I' : 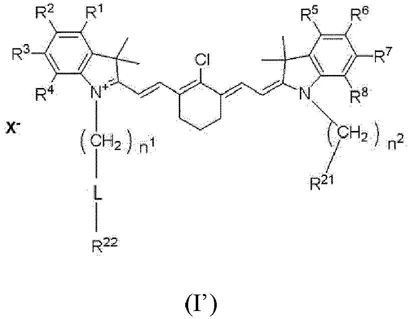 wherein,
X⁻ is anionic ion;
R¹-R⁸ are each independently H, hydroxyl, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, an optionally substituted C₃₋₁₀ cycloalkyl group, an optionally substituted 6-14-membered aryl group, an optionally substituted 5-14-membered heteroaryl group, and an optionally substituted 5-14-membered heterocyclic group; or R¹ and R², R² and R³ or R³ and R⁴ form an optionally substituted 3-8 membered carbon ring, 6-14 membered aromatic ring, 5-14 membered heteroaromatic ring or 5-14 membered hetero-ring with the carbon atoms to which they are attached, respectively, R⁵ and R⁶, R⁶ and R⁷ or R⁷ and R⁸ form an optionally substituted 3-8 membered carbon ring, 6-14 membered aromatic ring, 5-14 membered heteroaromatic ring or 5-14 membered hetero-ring with the carbon atoms to which they are attached, respectively;
R²¹ and R²² are each independently H, halogen, nitro, cyano, -SO₃, -COOH, -SO₃N(Rₐ)₃, C₁₋₆ alkyl, C₂₋₆ alkenyl, C₂₋₆ alkynyl, C₁₋₆ acyl, -NH₂, C₁₋₆ alkoxy, an optionally substituted C₃₋₁₅ cycloalkyl, an optionally substituted C₃₋₁₅ cycloalkyl C₁₋₆ alkyl, an optionally substituted 6-14-membered aryl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl; wherein each Rₐ is independently H and C₁₋₄ alkyl;
n¹ and n² are each independently selected from integers of 0-12, preferably integers of 0-6; and
L is a bond, or a linking group;
wherein the group -(CH₂)n¹-L-R²² is different from the group -(CH₂)n²-R²¹, and the steric hindrance of the group -(CH₂)n¹-L-R²² is greater than that of the group -(CH₂)n²-R²¹.

In one or more embodiments of the compound of Formula I' according to the present disclosure, R¹-R⁸ are all hydrogen.

In one or more preceding embodiments of the compound of Formula I' according to the present disclosure, L is selected from the group consisting of ^{∗}-T₁-C(O)-, *- C(O)-T₁-, *-S(O)₂-T₂- and ^{∗}-T₂-S(O)₂-, wherein T₁ is selected from the group consisting of NH, O and S, T₂ is selected from the group consisting of O, NH and S, * indicates the location in connection with -(CH₂)n¹-.

In one or more preceding embodiments of the compound of Formula I' according to the present disclosure, in R¹-R⁸ as well as R²¹ and R²², said an optionally substituted C₃₋₁₀ cycloalkyl, an optionally substituted 6-14-membered aryl, an optionally substituted 5-14-membered heteroaryl group, an optionally substituted 5-14-membered heterocyclic group, an optionally substituted C₃₋₁₅ cycloalkyl, an optionally substituted C₃₋₁₅ cycloalkyl C₁₋₆ alkyl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl are each an optionally substituted by 1-3 substitutents selected from the group consisting of halogen, C₁₋₆ alkyl, halogenated C₁₋₆ alkyl, hydroxyl, carboxyl, amino (including -NH₂ and -NR'R"), C₁₋₆ alkoxy and halogenated C₁₋₆ alkoxy, wherein R' is selected from C₁₋₄ alkyl and C₁₋₆ alkoxycarbonyl, and R" is selected from Hand C₁₋₄ alkyl.

In one or more preceding embodiments of the compound of Formula I' according to the present disclosure, R²² is C₆₋₁₄ aryl or C₃₋₁₅ cycloalkyl C₁₋₆ alkyl that is optionally substituted by 1 or 2 substitutents selected from the group consisting of halogen, C₁₋₆ alkyl, halogenated C₁₋₆ alkyl, amino, C₁₋₆ alkoxy and halogenated C₁₋₆ alkoxy.

In one or more preceding embodiments of the compound of Formula I' according to the present disclosure, R²² is C₆₋₁₄ aryl or C₃₋₁₅ cycloalkyl C₁₋₆ alkyl that is optionally substituted by 1 or 2 substitutents selected from the group consisting of halogen, C₁₋₆ alkyl, halogenated C₁₋₆ alkyl, -NH₂, -NR'R", C₁₋₆ alkoxy and halogenated C₁₋₆ alkoxy, wherein R' is selected from C₁₋₄ alkyl and C₁₋₆ alkoxycarbonyl, R" is selected from H and C₁₋₄ alkyl; R²¹ is H, halogen, C₁₋₆ alkyl, halogenated C₁₋₆ alkyl, C₂₋₆ alkenyl or C₂₋₆ alkynyl, and when R²¹ is H, n² is not 0. Preferably, R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl; the group -(CH₂)n²-R²¹ is C₁₋₆ alkyl or halogenated C₁₋₆ alkyl.

In one or more preceding embodiments of the compound of Formula I' according to the present disclosure, R¹-R⁸ are hydrogen; L is ^{∗}-T₁-C(O)- or *- C(O)-T₁-, wherein T₁ is selected from the group consisting of NH, O and S; n¹ and n² are each integer of 1 to 6; the group -(CH₂)n²-R²¹ is C₁₋₆ alkyl; R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from Hand C₁₋₄ alkyl.

In one or more preceding embodiments of the compound of Formula I' according to the present disclosure, the anion is selected from the group consisting of F⁻, Cl⁻, Br⁻, I⁻, NO₃⁻, SO₄²⁻, PO₄³⁻, HPO₄²⁻, H₂PO₄⁻, CO₃²⁻, HCO₃⁻, SO₃²⁻, HSO₃⁻, CH₃COO⁻ and CH₃SO₃⁻.

In one or more preceding embodiments of the compound of Formula I' according to the present disclosure, the compound of Formula I' has the structure represented by the following formula Ia', Ib' or Ic': 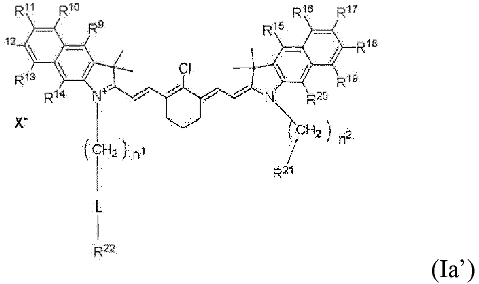 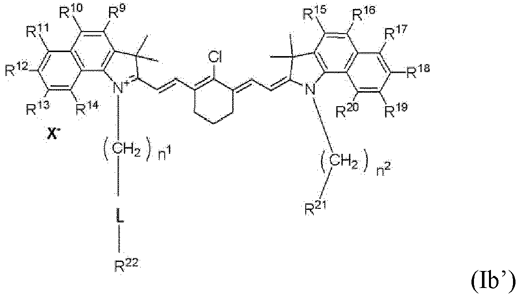 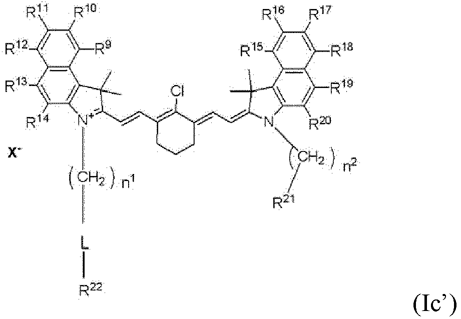
in each formula, n¹, n², L, R²¹ and R²² are as described in any of the foregoing embodiments of the compound according to the present disclosure;
R⁹-R²⁰ are each independently selected from the group consisting of H, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, C₃₋₁₀ cycloalkyl, C₆₋₁₄ aryl, C₅₋₁₄ heteroaryl and C₃₋₁₀ heterocyclic groups. Preferably, R⁹-R²⁰ are each independently H, halogen and C₁₋₆ alkyl, more preferably are all H.

In one or more preceding embodiments of the compound of Formula I' according to the present disclosure, the compound of Formula I' is selected from the group consisting of: 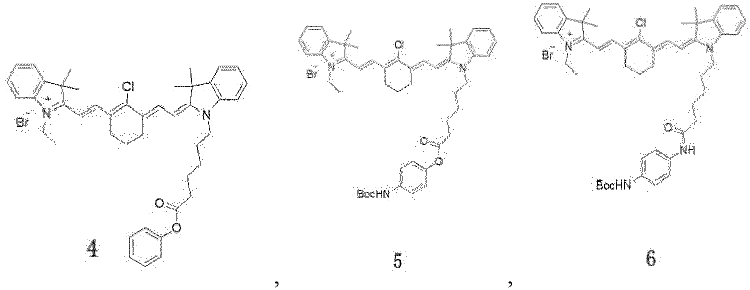 and

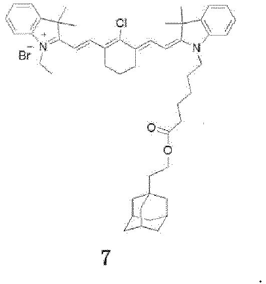

The present disclosure also provides a contrast agent comprising a compound of Formula I' according to the present disclosure and an optional anionic surfactant. Preferably, the anionic surfactant is selected from carboxylates, sulfonates, sulfate salts, phosphate ester salts and lipopeptide anionic surfactants; preferably, the anionic surfactant is selected from sulfonates and lipopeptide anionic surfactants; more preferably, the anionic surfactant is selected from the group consisting of surfactin, sodium lauryl sulfonate and sodium lauryl benzene sulfonate. Preferably, the final concentration of the compound of Formula I' in the contrast agent is in the range of 1 ~ 100 µM, such as 5 ~ 50 µM or 10 ~ 30 µM. Preferably, the contrast agent comprises anionic surfactant, and the molar ratio of the compound of Formula I' to the anionic surfactant in the contrast agent is 1: (0. 1-20), such as 1: (0.1-10) or 1: (0.2-5). Preferably, the contrast agent is a lyophilized powder or in the form of an aqueous solution.

The present disclosure also provides a detection kit comprising the compound of Formula I' according to the present disclosure and an optional anionic surfactant. Preferably, the anionic surfactant is as described in any embodiment herein, preferably surfactin or sodium dodecyl sulfonate (SDS). In some embodiments, the detection kit comprises the contrast agent described in any embodiment herein.

The present disclosure also provides use of the compound of Formula I' and the optional anionic surfactant as described in any embodiment herein in the manufacture of a tumor contrast agent.

The present disclosure also provides use of the compound of Formula I and the anionic surfactant as described in any embodiment herein in the manufacture of a tumor contrast agent.

The present disclosure also provides a tumor imaging method, which comprises a step of contacting a tissue to be examined with the contrast agent according to the present disclosure.

In one or more embodiments, the method is an in vitro method, said contacting comprises immersing, spraying and/or smearing the tissue to be examined with the contrast agent of the present disclsoure.

In one or more embodiments, the method is an in vivo method, said contacting comprises administering the contrast agent to the patient to be examined.

The present disclosure also provides a contrast agent or a kit containing the contrast agent, wherein the contrast agent comprises Compound 4 and/or Compound 6 and anionic surfactant: 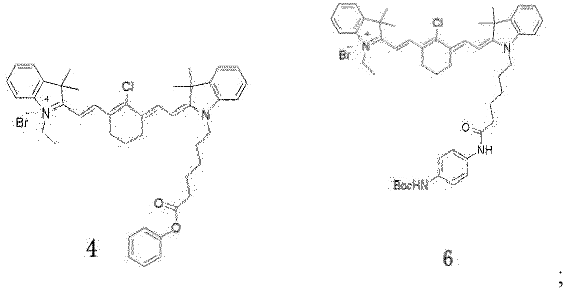 wherein the anionic surfactant is selected from the group consisting of surfactin, sodium lauryl sulfonate and sodium lauryl benzene sulfonate, and the molar ratio of Compound 4, Compound 6, or Compound 4 and Compound 6 to the anionic surfactant in the contrast agent is in the range of 1: (20-0.1), preferably 1: (10-0.1) or 1: (5-0.2). Preferably, the contrast agent is in the form of an aqueous solution or a lyophilized powder.

The present disclosure also provides a contrast agent or a kit containing the contrast agent, wherein the contrast agent comprises Compound 4 and/or Compound 6, and surfactin or sodium lauryl sulfonate: 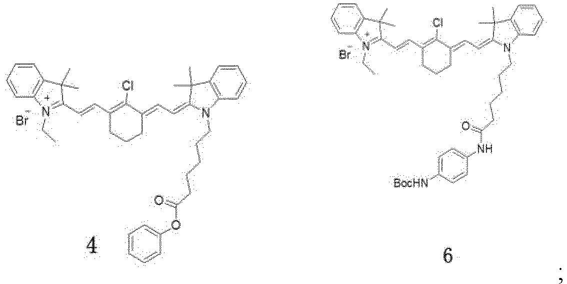 wherein the molar ratio of Compound 4, Compound 6, or Compound 4 and Compound 6 to surfactin or sodium lauryl sulfonate is in the range of 1: (20~0.1), preferably 1: (10∼0.1) or 1: (5-0.2).

Preferably, the contrast agent is in the form of an aqueous solution or a lyophilized powder.

### Description of the Drawings

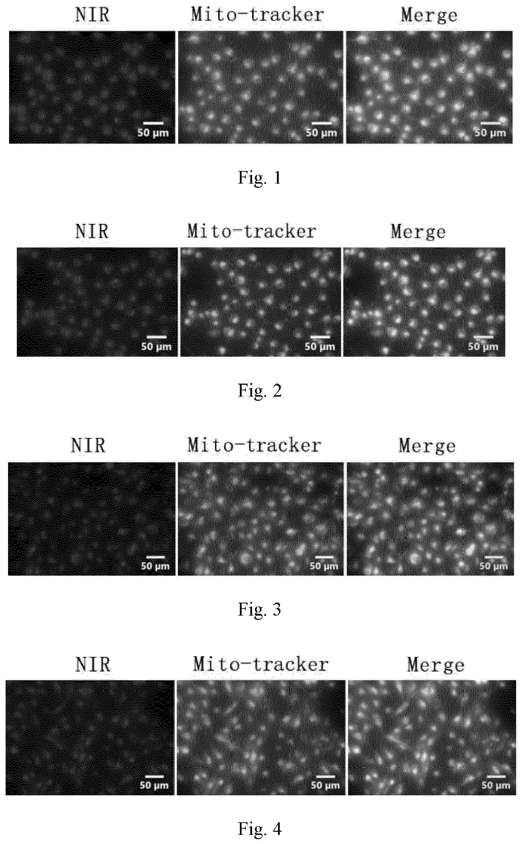

Fig. 1 is the result of fluorescence localization of Compound 1 in human renal cancer cell line 786o.
Fig. 2 is the result of fluorescence localization of Compound 2 in human renal cancer cell line 786o.
Fig. 3 is the result of fluorescence localization of Compound 3 in human renal cancer cell line 786o.
Fig. 4 is the result of fluorescence localization of Compound 4 in human renal cancer cell line 786o.

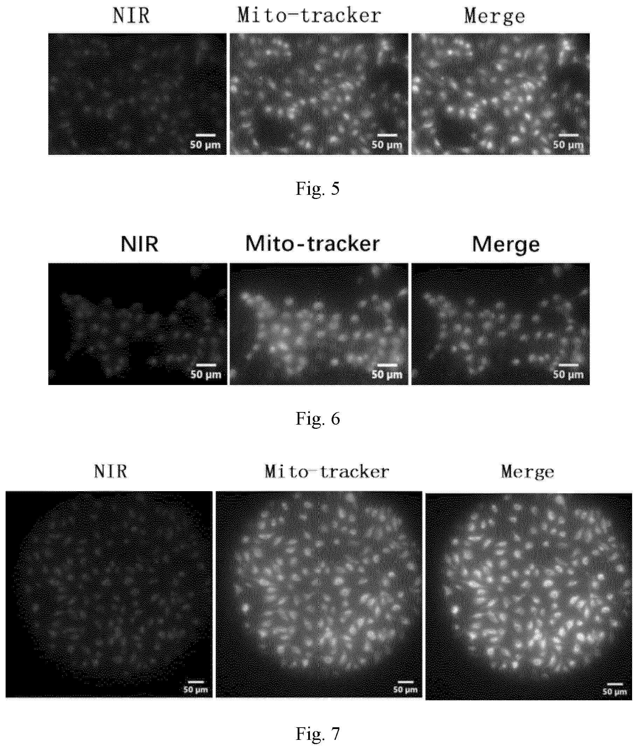

Fig. 5 is the result of fluorescence localization of Compound 5 in human renal cancer cell line 786o.
Fig. 6 is the result of fluorescence localization of Compound 7 in human renal cancer cell line 786o.
Fig. 7 is the result of fluorescence localization of Compound 4 in human renal cancer cell line A498.

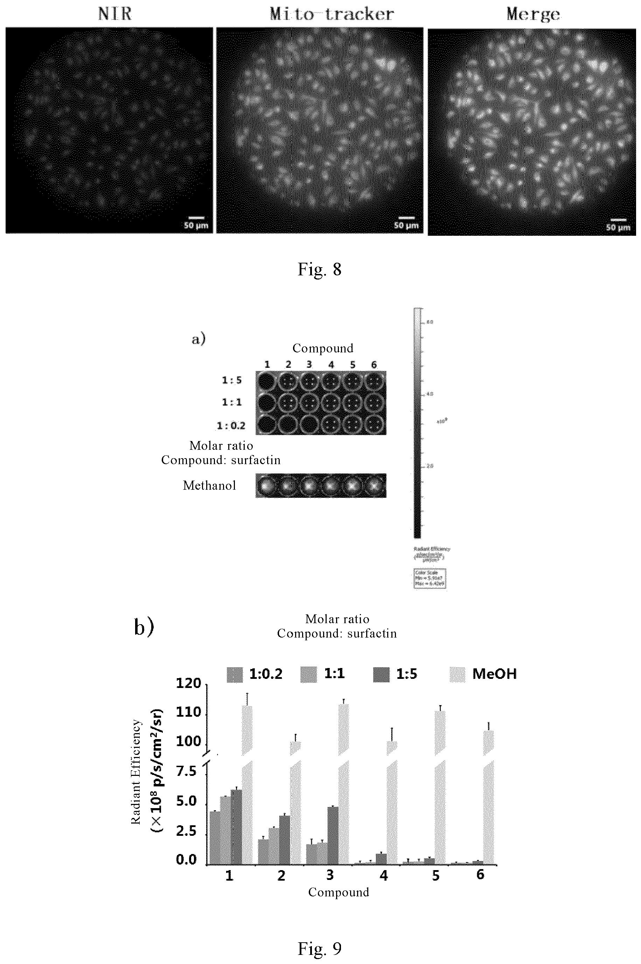

Fig. 8 is the result of fluorescence localization of Compound 5 in human renal cancer cell line A498.
Fig. 9 is the result of fluorescence imaging results (a) and ROI numerical quantitative analysis (b) after mixing Compounds 1-5 and 7 with surfactin in different ratios.

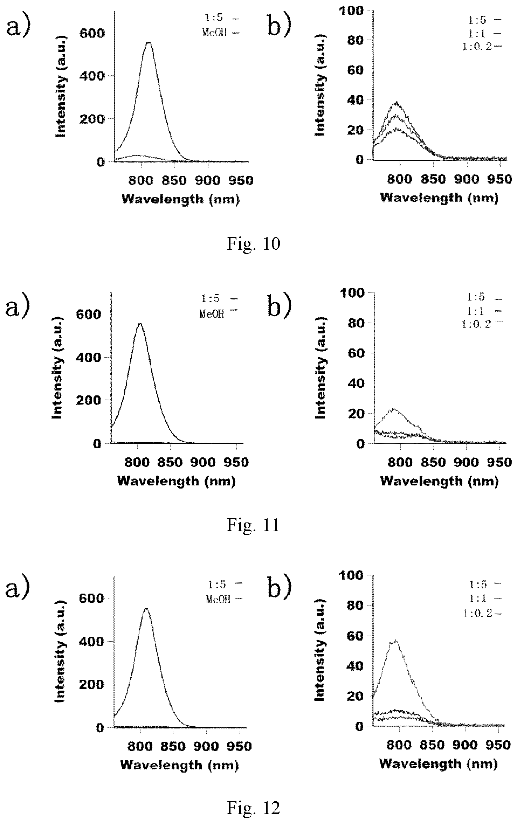

Fig.10 is the fluorescence emission spectra of Compound 1 mixed with surfactin in different ratios (Ex=730 nm, Em=760-960 nm): (a) fluorescence emission spectra of an aqueous solution of Compound 1 and surfactin with a molar ratio of 1:5 and a methanol solution of Compound 1; (b) fluorescence emission spectra of Compound 1 and surfactin with a molar ratio of 1:5, 1:1, 1:0.2.
Fig.11 is the fluorescence emission spectra of Compound 2 mixed with surfactin in different ratios (Ex=730 nm, Em=760-960 nm): (a) fluorescence emission spectra of an aqueous solution of Compound 2 and surfactin with a molar ratio of 1:5 and a methanol solution of Compound 1; (b) fluorescence emission spectra of Compound 2 and surfactin with a molar ratio of 1:5, 1:1, 1:0.2.
Fig.12 is the fluorescence emission spectra of Compound 3 mixed with surfactin in different ratios (Ex=730 nm, Em=760-960 nm): (a) fluorescence emission spectra of an aqueous solution of Compound 3 and surfactin with a molar ratio of 1:5 and a methanol solution of Compound 1; (b) fluorescence emission spectra of Compound 3 and surfactin with a molar ratio of 1:5, 1:1, 1:0.2.

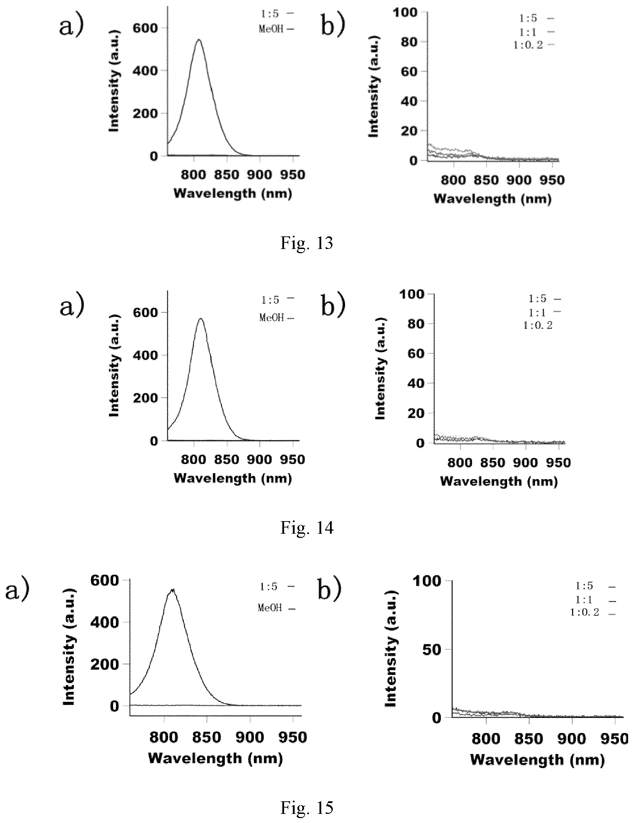

Fig.13 is the fluorescence emission spectra of Compound 4 mixed with surfactin as anionic surfactant in different ratios (Ex=730 nm, Em=760-960 nm): (a) fluorescence emission spectra of an aqueous solution of Compound 4 and surfactin with a molar ratio of 1:5 and a methanol solution of Compound 1; (b) fluorescence emission spectra of Compound 4 and surfactin with a molar ratio of 1:5, 1:1, 1:0.2.
Fig.14 is the fluorescence emission spectra of Compound 5 mixed with surfactin in different ratios (Ex=730 nm, Em=760-960 nm): (a) fluorescence emission spectra of an aqueous solution of Compound 5 and surfactin with a molar ratio of 1:5 and a methanol solution of Compound 1; (b) fluorescence emission spectra of Compound 5 and surfactin with a molar ratio of 1:5, 1:1, 1:0.2.
Fig.15 is the fluorescence emission spectra of Compound 6 mixed with surfactin in different ratios (Ex=730 nm, Em=760-960 nm): (a) fluorescence emission spectra of an aqueous solution of Compound 6 and surfactin with a molar ratio of 1:5 and a methanol solution of Compound 1; (b) fluorescence emission spectra of Compound 6 and surfactin with a molar ratio of 1:5, 1:1, 1:0.2.

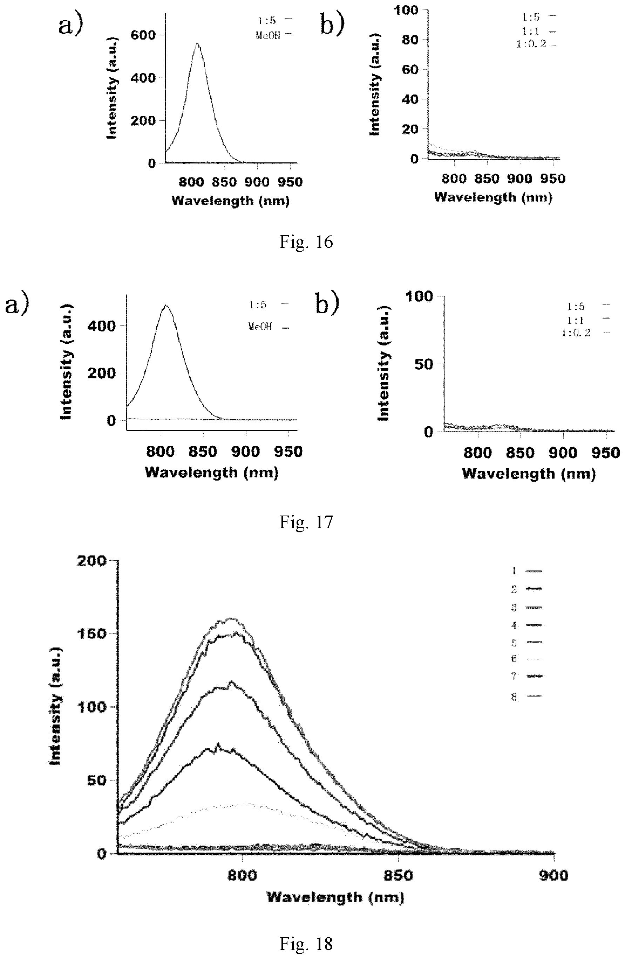

Fig.16 is the fluorescence emission spectra of Compound 7 mixed with surfactin in different ratios (Ex=730 nm, Em=760-960 nm): (a) fluorescence emission spectra of an aqueous solution of Compound 7 and surfactin with a molar ratio of 1:5 and a methanol solution of Compound 1; (b) fluorescence emission spectra of Compound 7 and surfactin with a molar ratio of 1:5, 1:1, 1:0.2.
Fig.17 is the fluorescence emission spectra of Compound 8 mixed with surfactin in different ratios (Ex=730 nm, Em=760-960 nm): (a) fluorescence emission spectra of an aqueous solution of Compound 8 and surfactin with a molar ratio of 1:5 and a methanol solution of Compound 1; (b) fluorescence emission spectra of Compound 8 and surfactin with a molar ratio of 1:5, 1:1, 1:0.2.
Fig.18 is the fluorescence emission spectra of Compound 4 mixed with various surfactants (Ex=730 nm, Em=760-960 nm). The specific surfactants are as follows: 1 is surfactin; 2 is human serum albumin; 3 is polyoxyethylene castor oil (Kolliphor); 4 is polyethylene oxide-polypropylene oxide-polyethylene oxide triblock copolymer (P123); 5 is dodecyltrimethylammonium chloride; 6 is tetrabutylammonium iodide; 7 is sodium dodecyl sulfonate; 8 is sodium dodecylbenzenesulfonate.

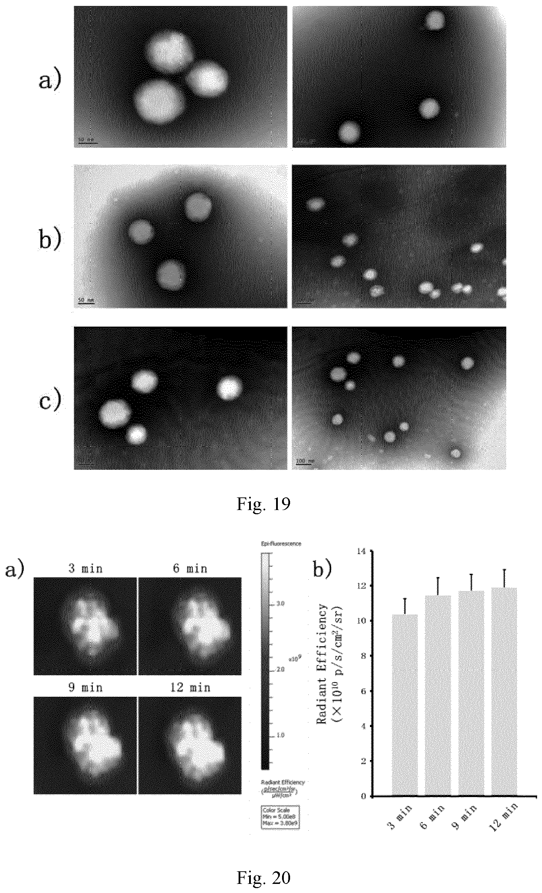

Fig.19 is the result of transmission electron microscopy test: (a) Compound 4; (b) Compound 5; (c) Compound 7.
Fig.20 is the near-infrared imaging results of tumor tissue in a mouse subcutaneous xenograft renal clear cell carcinoma tumor model in a mixture of Compound 1 and surfactin as anionic surfactant (the molar ratio of the compound to surfactin is 1:5): (a) typical pictures of near-infrared imaging results; (b) results of ROI numerical quantitative analysis of tumor tissue.

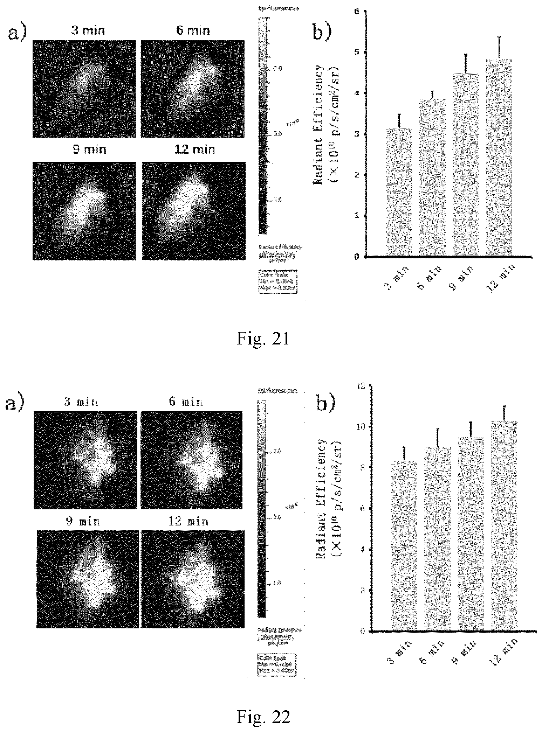

Fig.21 is the near-infrared imaging results of tumor tissue in a mouse subcutaneous xenograft renal clear cell carcinoma tumor model in a mixture of Compound 2 and surfactin as anionic surfactant (the molar ratio of the compound to surfactin is 1:5): (a) typical pictures of near-infrared imaging results; (b) results of ROI numerical quantitative analysis of tumor tissue.
Fig.22 is the near-infrared imaging results of tumor tissue in a mouse subcutaneous xenograft renal clear cell carcinoma tumor model in a mixture of Compound 3 and surfactin as anionic surfactant (the molar ratio of the compound to surfactin is 1:5): (a) typical pictures of near-infrared imaging results; (b) results of ROI numerical quantitative analysis of tumor tissue.

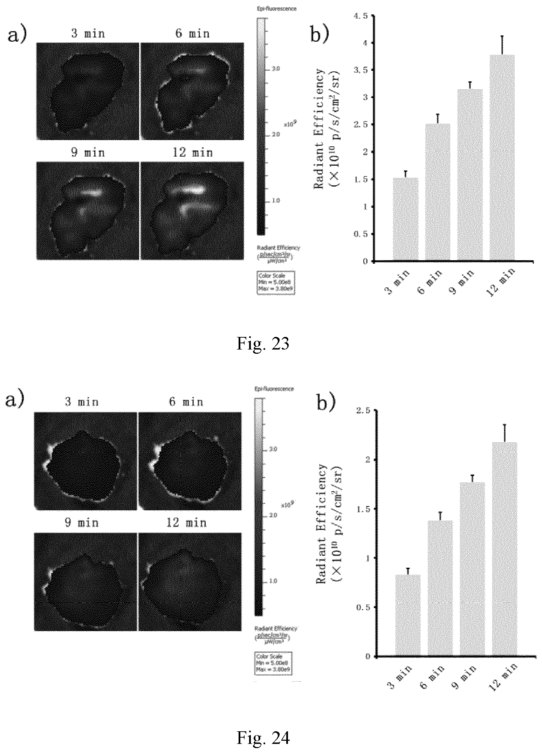

Fig.23 is the near-infrared imaging results of tumor tissue in a mouse subcutaneous xenograft renal clear cell carcinoma tumor model in a mixture of Compound 4 and surfactin as anionic surfactant (the molar ratio of the compound to surfactin is 1:5): (a) typical pictures of near-infrared imaging results; (b) results of ROI numerical quantitative analysis of tumor tissue.
Fig.24 is the near-infrared imaging results of tumor tissue in a mouse subcutaneous xenograft renal clear cell carcinoma tumor model in a mixture of Compound 5 and surfactin as anionic surfactant (the molar ratio of the compound to surfactin is 1:5): (a) typical pictures of near-infrared imaging results; (b) results of ROI numerical quantitative analysis of tumor tissue.

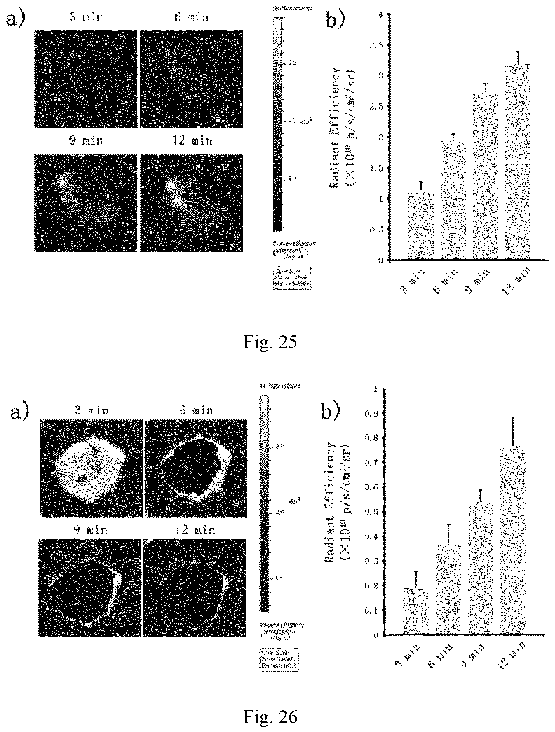

Fig.25 is the near-infrared imaging results of tumor tissue in a mouse subcutaneous xenograft renal clear cell carcinoma tumor model in a mixture of Compound 6 and surfactin as anionic surfactant (the molar ratio of the compound to surfactin is 1:5): (a) typical pictures of near-infrared imaging results; (b) results of ROI numerical quantitative analysis of tumor tissue.
Fig.26 is the near-infrared imaging results of tumor tissue in a mouse subcutaneous xenograft renal clear cell carcinoma tumor model in a mixture of Compound 7 and surfactin as anionic surfactant (the molar ratio of the compound to surfactin is 1:5): (a) typical pictures of near-infrared imaging results; (b) results of ROI numerical quantitative analysis of tumor tissue.

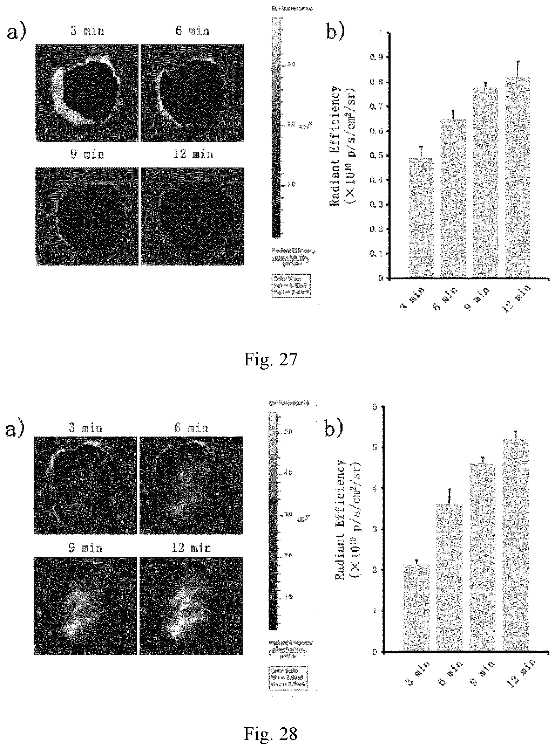

Fig.27 is the near-infrared imaging results of tumor tissue in a mouse subcutaneous xenograft renal clear cell carcinoma tumor model in a mixture of Compound 8 and surfactin as anionic surfactant (the molar ratio of the compound to surfactin is 1:5): (a) typical pictures of near-infrared imaging results; (b) results of ROI numerical quantitative analysis of tumor tissue.
Fig.28 is the near-infrared imaging results of tumor tissue in a mouse subcutaneous xenograft breast cancer tumor model in a mixture of Compound 4 and surfactin as anionic surfactant (the molar ratio of the compound to surfactin is 1:5): (a) typical pictures of near-infrared imaging results; (b) results of ROI numerical quantitative analysis of tumor tissue.

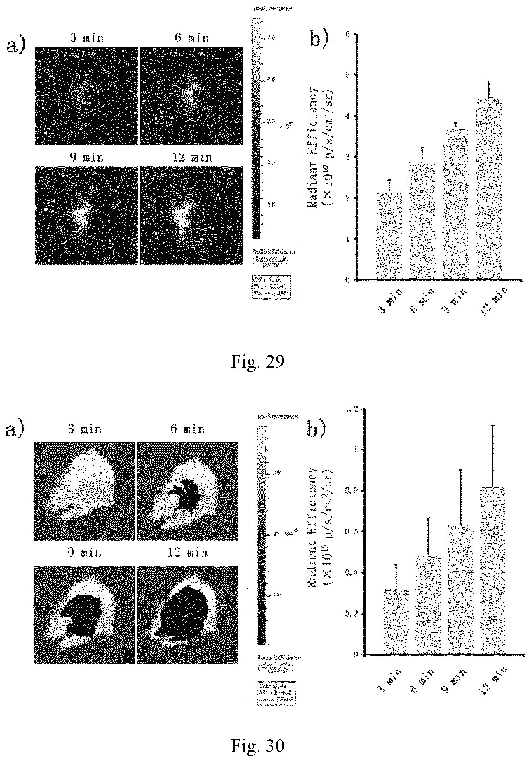

Fig.29 is the near-infrared imaging results of tumor tissue in a mouse subcutaneous xenograft breast cancer tumor model in a mixture of Compound 6 and surfactin as anionic surfactant (the molar ratio of the compound to surfactin is 1:5): (a) typical pictures of near-infrared imaging results; (b) results of ROI numerical quantitative analysis of tumor tissue.
Fig.30 is the near-infrared imaging results of tumor tissue in a mouse subcutaneous xenograft renal clear cell carcinoma tumor model in a mixture of Compound 4 and surfactin as anionic surfactant (the molar ratio of the compound to surfactin is 1:5) and 50-fold sulfobromophthalein sodium competition experiment: (a) typical pictures of near-infrared imaging results; (b) results of ROI numerical quantitative analysis of tumor tissue.

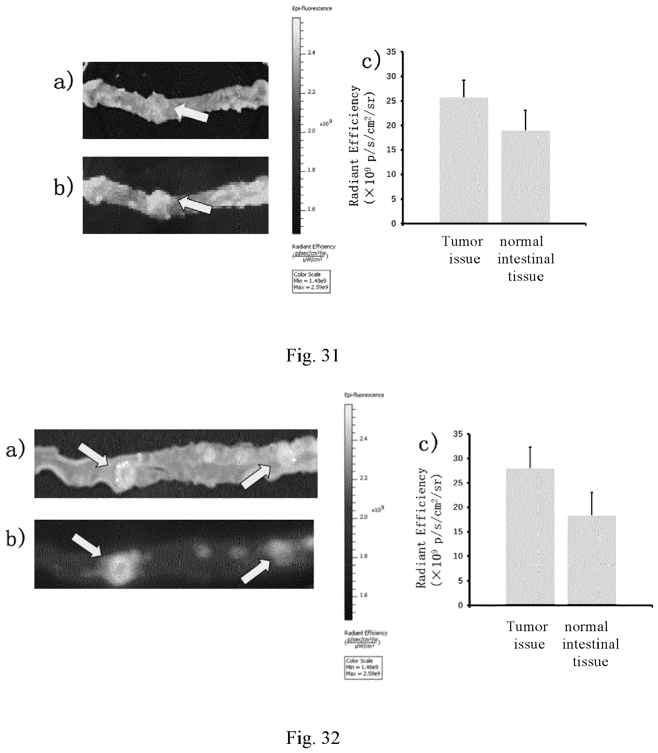

Fig.31 is the near-infrared imaging results of diseased colonic tissue in a mouse primary bowel cancer tumor model in a mixture of Compound 1 and surfactin as anionic surfactant (the molar ratio of the compound to surfactin is 1:5): (a) imaging results in visible light (the yellow arrows refers to the tumor tissue); (b) infrared imaging results (the yellow arrow refers to the tumor tissue); (c) results of ROI numerical quantitative analysis of tumor tissue and normal bowel tissue.
Fig.32 is the near-infrared imaging results of diseased colonic tissue in a mouse primary bowel cancer tumor model in a mixture of Compound 2 and surfactin as anionic surfactant (the molar ratio of the compound to surfactin is 1:5): (a) imaging results in visible light (the yellow arrows refers to the tumor tissue); (b) infrared imaging results (the yellow arrow refers to the tumor tissue); (c) results of ROI numerical quantitative analysis of tumor tissue and normal bowel tissue.

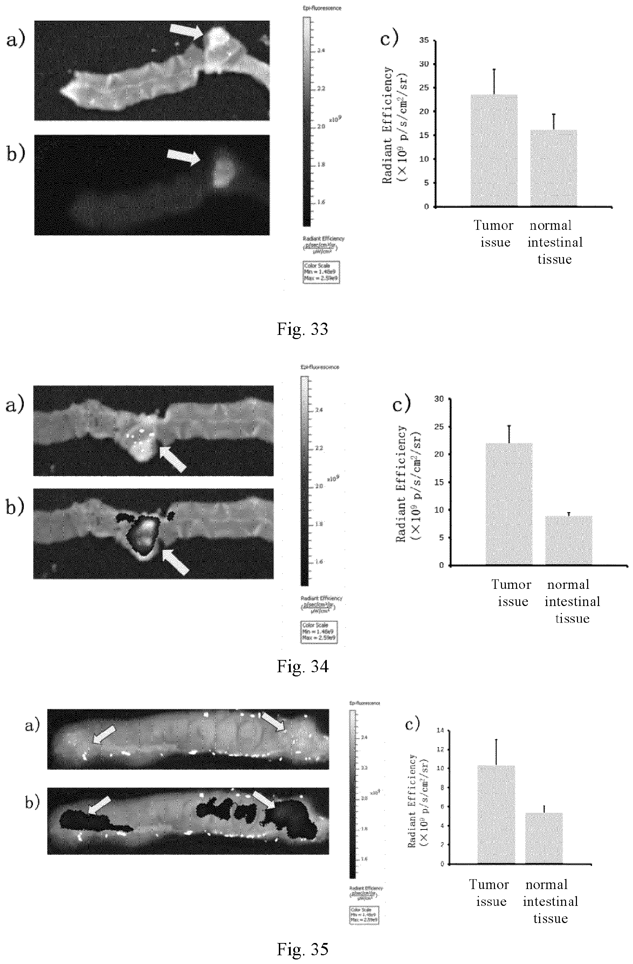

Fig.33 is the near-infrared imaging results of diseased colonic tissue in a mouse primary bowel cancer tumor model in a mixture of Compound 3 and surfactin as anionic surfactant (the molar ratio of the compound to surfactin is 1:5): (a) imaging results in visible light (the yellow arrows refers to the tumor tissue); (b) infrared imaging results (the yellow arrow refers to the tumor tissue); (c) results of ROI numerical quantitative analysis of tumor tissue and normal bowel tissue.
Fig.34 is the near-infrared imaging results of diseased colonic tissue in a mouse primary bowel cancer tumor model in a mixture of Compound 4 and surfactin as anionic surfactant (the molar ratio of the compound to surfactin is 1:5): (a) imaging results in visible light (the yellow arrows refers to the tumor tissue); (b) infrared imaging results (the yellow arrow refers to the tumor tissue); (c) results of ROI numerical quantitative analysis of tumor tissue and normal bowel tissue.
Fig.35 is the near-infrared imaging results of diseased colonic tissue in a mouse primary bowel cancer tumor model in a mixture of Compound 5 and surfactin as anionic surfactant (the molar ratio of the compound to surfactin is 1:5): (a) imaging results in visible light (the yellow arrows refers to the tumor tissue); (b) infrared imaging results (the yellow arrow refers to the tumor tissue); (c) results of ROI numerical quantitative analysis of tumor tissue and normal bowel tissue.

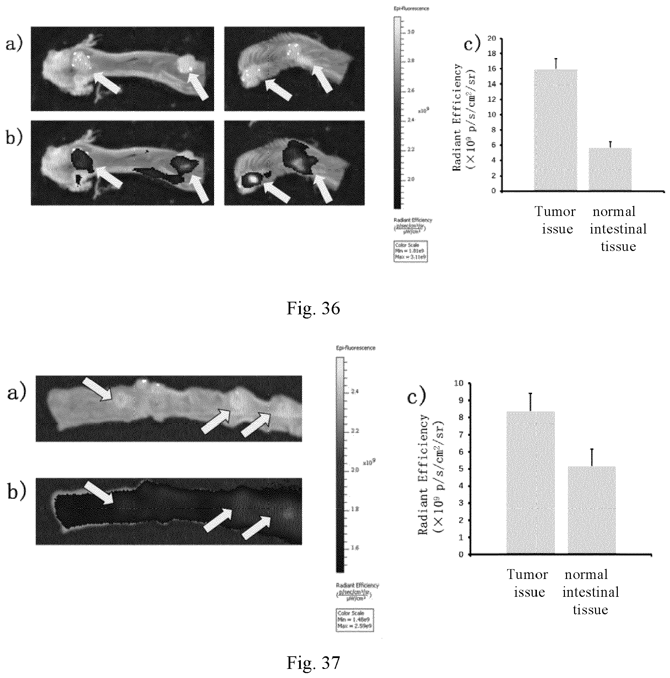

Fig.36 is the near-infrared imaging results of diseased colonic tissue in a mouse primary bowel cancer tumor model in a mixture of Compound 6 and surfactin as anionic surfactant (the molar ratio of the compound to surfactin is 1:5): (a) imaging results in visible light (the yellow arrows refers to the tumor tissue); (b) infrared imaging results (the yellow arrow refers to the tumor tissue).
Fig.37 is the near-infrared imaging results of diseased colonic tissue in a mouse primary bowel cancer tumor model in a mixture of Compound 7 and surfactin as anionic surfactant (the molar ratio of the compound to surfactin is 1:5): (a) imaging results in visible light (the yellow arrows refers to the tumor tissue); (b) infrared imaging results (the yellow arrow refers to the tumor tissue).
Fig.38 is the near-infrared imaging results of diseased colonic tissue in a mouse primary bowel cancer tumor model in a mixture of Compound 8 and surfactin as anionic surfactant (the molar ratio of the compound to surfactin is 1:5): (a) imaging results in visible light (the yellow arrows refers to the tumor tissue); (b) infrared imaging results (the yellow arrow refers to the tumor tissue).
Fig. 39 is the schematic diagram of the working principle of the contrast agent of the present disclosure.

### Detailed Description of the Disclosure

It should be understood that within the scope of the present disclosure, the above technical features of the present disclosure and the technical features specifically described below (such as in examples) may be combined with each other, thereby constituting a preferred technical solution.

### I. Terms and definitions

The theory or mechanism described and disclosed herein, whether right or wrong, should not limit the scope of the disclosure in any way, i.e., the content of the disclosure may be implemented without being limited by any particular theory or mechanism.

The range of values described herein shall be deemed to have covered and specifically disclosed all possible sub-ranges and any individual values within the range. For example, "comprising 1 to 20 carbon atoms" would include comprising 1 to 10 carbon atoms, comprising 2 to 10 carbon atoms, comprising 5 carbon atoms, etc.

Herein, when describing embodiments, or examples, it should be understood that it is not intended to limit the present disclosure to these embodiments or examples. In contrast, all substitutes, modifications and equivalents of the methods and materials described in the present disclosure may be covered within the scope of the claims. Those skilled in the art will be able to identify a number of methods and materials similar to or equivalent to those described herein that may be used to implement the present disclosure.

In the present disclosure, for the sake of conciseness, not all possible combinations of various technical features in various embodiments or examples are described. Thus, as long as there is no contradiction in the combination of these technical features, each technical feature of each embodiment or example may be arbitrarily combined, and all possible combinations should be considered to be within the scope of the present description.

In the present disclosure, halogens include F, Cl, Br, and I.

The term "alkyl" refers to a linear or branched monovalent saturated hydrocarbon group, typically containing 1-12 carbon atoms (C₁₋₁₂ alkyl), preferably 1-6 carbon atoms (C₁₋₆ alkyl). Examples of alkyl groups include, but are not limited to, methyl, ethyl, n-propyl, isopropyl, n-butyl, isobutyl, and tert-butyl. Alkylene refers to divalent alkyls are such as -CH₂-, - CH₂CH₂-, etc. The number of carbon atoms of alkylene group is usually 1-6.

The term "alkenyl" refers to a straight-chain or branched monovalent hydrocarbon group containing one or more double bonds, typically containing 2-12 carbon atoms (C₂₋₁₂ alkenyl), preferably containing 2-6 carbon atoms (C₂₋₆ alkenyl). Examples of alkenyl include, but are not limited to, vinyl, propenyl, allyl and 1,4-butadienyl.

The term "alkynyl" refers to a straight or branched monovalent hydrocarbon group containing one or more triple bonds, typically containing 2-12 carbon atoms (C₂₋₁₂ alkynyl), preferably containing 2-6 carbon atoms (C₂₋₆ alkynyl). Examples of alkynyl include, but are not limited to, ethynyl, 1-propynyl, 1-butynyl, 2-butynyl, and 1-methyl-2-butynyl.

The term "cyclic hydrocarbon group" or "carbon ring group" means a stable non-aromatic monocyclic or polycyclic hydrocarbon group composed only of carbon atoms and hydrogen atoms, which may include a fused ring system, a bridge ring system or a spiro ring system having 3 to 15 carbon atoms, preferably 3 to 10 carbon atoms, and which are saturated or unsaturated and may be connected to the rest of the molecule via any suitable carbon atom by a single bond. Unless otherwise specified in the specification, carbon atoms in cyclic hydrocarbon groups may optionally be oxidized. Examples of cyclic hydrocarbon groups include, but are not limited to, cyclopropyl, cyclobutyl, cyclopentyl, cyclopentenyl, cyclohexyl, cyclohexadienyl, cycloheptyl, cyclooctyl, 1H-indenyl, indanyl, 1,2,3,4-tetrahydro-naphthyl, 5,6,7,8-tetrahydro-naphthyl, 8,9-dihydro-7H-benzocyclohepten-6-yl, 6,7,8,9-tetrahydro-5H-benzocycloheptenyl, 5,6,7,8,9,10-hexahydro-benzocyclooctenyl, fluorenyl, dicyclo[2.2.1]heptyl, 7,7-dimethyl-dicyclo[2.2.1]heptyl, dicyclo[2.2.1]heptenyl, dicyclo[2.2.2]octyl, dicyclo[3.1.1]heptyl, dicyclo[3.2.1]octyl, dicyclo[2.2.2]octenyl, dicyclo[3.2.1]octenyl, adamantyl, octahydro-4,7-methylene-1H-indenyl and octahydro-2,5-methylene-cyclopentadienyl, etc. Exemplary cyclic hydrocarbon groups or carbon ring groups are cycloalkyl groups usually having a number of carbon atoms in the ring of 3-10, preferably 3-8. Examples of cycloalkyl include, but are not limited to, cyclopropyl, cyclobutyl, cyclopentyl, cyclohexyl, cycloheptyl, cyclooctyl and adamantyl. Exemplary cyclic hydrocarbon groups or carbon ring groups can be cycloalkenyl groups, i.e. monovalent hydrocarbon groups containing non-aromatic double-bonded hydrocarbon rings. The number of carbon atoms in the ring of the cycloalkenyl group is usually 3-10, such as 3-8. Examples of cycloalkenyl group include, but are not limited to, cyclopentenyl, cyclohexenyl, and cycloheptenyl.

The term "aryl" or "aromatic ring" refers to a monovalent hydrocarbon group containing an aromatic hydrocarbon ring and is usually C6-14 aryl. Examples of aryl groups include, but are not limited to, phenyl, naphthyl, and anthracenyl.

The term "heteroaryl" or "heteroaromatic ring" refers to a group containing 5-14 ring atoms and 6, 10 or 14 electrons that are shared on the ring system, wherein the ring atoms contained are carbon atoms and 1-3 heteroatoms selected from the group consisting of oxygen, nitrogen, and sulfur. Examples of heteroaryl groups include, but are not limited to, thienyl, benzisothiazolyl, benzothienyl, naphtho[2,3-b]thienyl, thianthrenyl, furanyl, pyranyl, isobenzofuranyl, chromenyl, xanthenyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinazinyl, isoquinolinyl, quinolinyl, phthalazinyl, naphthyridinyl, quinazolinyl, cinnolinyl, pteridinyl, carbazolyl, β-carbolinyl, phenanthridinyl, acridinyl, perimidinyl, phenanthrolinyl, phenazinyl, isothiazolyl, phenothiazinyl and isoxazolyl, etc.

The term "heterocyclic group" or "hetero-ring" refers to a saturated or partially saturated 3-7-membered single ring or 7-10-membered bicyclic system consisting of carbon atoms and 1-3 heteroatoms selected from the group consisting of O, N, and S. Heterocyclic groups include, but are not limited to, tetrahydrofuranyl, pyrranyl, piperidinyl, piperazinyl, pyrrolidinyl, imidazolidyl, imidazolidinyl, dihydroindolyl, isodihydroindolyl, quinuclidinyl, morpholinyl, isochromanyl, chromanyl, pyrazolidinyl and pyrazolinyl and the like.

The groups described herein may be substituted. The number of substituents can be one or more, for example 1-4. Unless otherwise indicated, when a group is referred to herein as being "an optionally substituted", its substituents typically include, but are not limited to, one or more (for example, 1, 2, 3 or 4) of halogen, C₁₋₆ alkyl, halogenated C₁₋₆ alkyl, C₁₋₆ alkoxy, halogenated C₁₋₆ alkoxy, C₂₋₆ alkenyl, C₂₋₆ alkynyl, C₆₋₁₄ aryl, C₅₋₁₄ heteroaryl, C₃₋₁₀ heterocyclic group, C₃₋₈ cycloalkyl, hydroxyl, nitro, cyano, mercapto, amino, C₁₋₆ acyl. In a preferred embodiment, the substituent is one or more selected from the group consisting of halogen, hydroxyl, amino, C₁₋₆ alkyl and C₁₋₆ alkoxy.

In the present disclosure, "amino" includes -NH₂ and -NR'R", wherein R' and R" are each independently H, C₁₋₄ alkyl and C₁₋₆ alkoxycarbonyl; preferably, R' is selected from C₁₋₄ alkyl and C₁₋₆ alkoxycarbonyl, R" is selected from Hand C₁₋₄ alkyl.

### II. Contrast agents and detection kits

The contrast agent of the present disclosure comprises one or more compounds represented by Formula I: 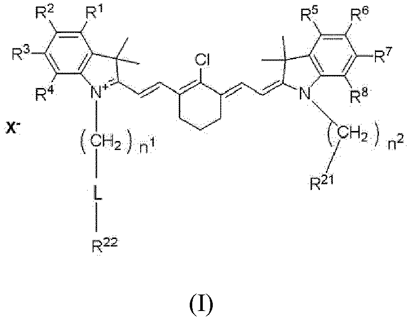 wherein,
X⁻ is anionic ion;
R¹-R⁸ are each independently H, hydroxyl, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, an optionally substituted C₃₋₁₀ cycloalkyl group, an optionally substituted 6-14-membered aryl group, an optionally substituted 5-14-membered heteroaryl group, and an optionally substituted 5-14-membered heterocyclic group; or R¹ and R², R² and R³ or R³ and R⁴ form an optionally substituted 3-8 membered carbon ring, 6-14 membered aromatic ring, 5-14 membered heteroaromatic ring or 5-14 membered hetero-ring with the carbon atoms to which they are attached, respectively, R⁵ and R⁶, R⁶ and R⁷ or R⁷ and R⁸ form an optionally substituted 3-8 membered carbon ring, 6-14 membered aromatic ring, 5-14 membered heteroaromatic ring or 5-14 membered hetero-ring with the carbon atoms to which they are attached, respectively;
R²¹ and R²² are each independently H, halogen, nitro, cyano, -SO₃, -COOH, -SO₃N(Rₐ)₃, C₁₋₆ alkyl, C₂₋₆ alkenyl, C₂₋₆ alkynyl, C₁₋₆ acyl, -NH₂, C₁₋₆ alkoxy, an optionally substituted C₃₋₁₅ cycloalkyl, an optionally substituted C₃₋₁₅ cycloalkyl C₁₋₆ alkyl, an optionally substituted 6-14-membered aryl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl; wherein each Rₐ is independently H and C₁₋₄ alkyl;
n¹ and n² are each independently selected from integers of 0-12, preferably integers of 0-6; and
L is a bond, or a linking group.

In some embodiments of the compound of Formula I, L is the bond, n¹ and n² are the same integers, and R²¹ and R²² are the same groups. Therefore, in these embodiments, the group -(CH₂)n¹-L-R²² is the same as the group -(CH₂)n²-R²¹, for example, both of which are C₁₋₆ alkyl groups that are optionally substituted by 1 or 2 substituents selected from the group consisting of halogen, nitro, cyano, -SO₃, -COOH and -NH₂. Preferably, the group -(CH₂)n¹-L-R²² is the same as the group -(CH₂)n²-R²¹, and both are C₁₋₆ alkyl groups that are optionally substituted by -COOH. More preferably, in these embodiments, R¹-R⁸ are all hydrogen.

In some other embodiments of the compound of Formula I, the group -(CH₂)n¹-L-R²² is different from the group -(CH₂)n²-R²¹, and the steric hindrance of the group -(CH₂)n¹-L-R²² is greater than that of the group -(CH₂)n²-R²¹. For example, R²² is selected from the group consisting of an optionally substituted C₃₋₁₅ cycloalkyl, an optionally substituted C₃₋₁₅ cycloalkyl C₁₋₆ alkyl, an optionally substituted 6-14-membered aryl group, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl, while R²¹ is not selected from these groups, for example, R²¹ is H, halogen, nitro, cyano, -SO₃, -COOH, C₁₋₆ alkyl, C₂₋₆ alkenyl, C₂₋₆ alkynyl, C₁₋₆ acyl, -NH₂ or C₁₋₆ alkoxy, preferably H, C₁₋₆ alkyl, C₂₋₆ alkenyl, C₂₋₆ alkynyl, C₁₋₆ acyl, -NH₂ or C₁₋₆ alkoxy, more preferably H or C₁₋₆ alkyl. Preferably, in these embodiments, R¹-R⁸ are all hydrogen. Preferably, in these embodiments, L is selected from the group consisting of ^{∗}-T₁-C(O)-, *- C(O)-T₁-, ^{∗}-S(O)₂-T₂- and ^{∗}-T₂-S(O)₂-, wherein T₁ is selected from the group consisting of NH, O and S, T₂ is selected from the group consisting of O, NH and S, * indicates the location in connection with -(CH₂)n¹-. More preferably, L is selected from ^{∗}-T₁-C(O)- and *- C(O)-T₁-, wherein T₁ is selected from NH and O.

In some embodiments, the group -(CH₂)n¹-L-R²² is different from the group -(CH₂)n²-R²¹, and the group -(CH₂)n¹-L-R²² is C₁₋₆ alkyl that is optionally substituted by 1 or 2 substitutents selected from the group consisting of halogen, nitro, cyano, -SO₃, -COOH, - NH₂, preferably C₁₋₆ alkyl that is optionally substituted by -COOH; the group -(CH₂)n²-R²¹ is C₁₋₆ alkyl that is optionally substituted by 1 or 2 substitutents selected from the group consisting of halogen, nitro, cyano, -SO₃, -COOH, -NH₂, preferably C₁₋₆ alkyl that is optionally substituted by -COOH, and the carbon chain length of the group -(CH₂)n²-R²¹ is shorter than the carbon chain length of the group -(CH₂)n¹-L-R²². Preferably, the group - (CH₂)n¹-L-R²² is C₁₋₆ alkyl that is optionally substituted by -COOH, the group -(CH₂)n²-R²¹ is C₁₋₆ alkyl that is optionally substituted by -COOH, and the carbon chain length of the group -(CH₂)n²-R²¹ is shorter than the carbon chain length of the group -(CH₂)n¹-L-R²². More preferably, the group -(CH₂)n¹-L-R²² is C₁₋₆ alkyl that is substituted by -COOH, the group -(CH₂)n²-R²¹ is an unsubstituted C₁₋₆ alkyl, and the carbon chain length of the group -(CH₂)n²-R²¹ is shorter than the carbon chain length of the group -(CH₂)n¹-L-R²². Preferably, in these embodiments, L is a bond.

Preferably, the compound of Formula I does not comprise Compound 8.

Preferably, in the compound of Formula I, R₁-R₈ are each independently H, halogen or C₁-C₄ alkyl.

Preferably, in the compound of Formula I, L is selected from the group consisting of *-T₁-C(O)-, *- C(O)-T₁-, ^{∗}-S(O)₂-T₂- and ^{∗}-T₂-S(O)₂-, wherein T₁ is selected from the group consisting of NH, O and S, T₂ is selected from the group consisting of O, NH and S, * indicates the location in connection with -(CH₂)n¹-. More preferably, L is ^{∗}-T₁-C(O)- or *-C(O)-T₁-, T₁ is preferably NH or O.

Preferably, in Formula I, in R¹-R⁸ as well as R²¹ and R²², said an optionally substituted C₃₋₁₀ cycloalkyl, an optionally substituted 6-14-membered aryl, an optionally substituted 5-14-membered heteroaryl group, an optionally substituted 5-14-membered heterocyclic group, an optionally substituted C₃₋₁₅ cycloalkyl, an optionally substituted C₃₋₁₅ cycloalkyl C₁₋₆ alkyl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl are each an optionally substituted by 1-3 substitutents selected from the group consisting of halogen, C₁₋₆ alkyl, halogenated C₁₋₆ alkyl, hydroxyl, carboxyl, amino (including -NH₂ and -NR'R"), C₁₋₆ alkoxy and halogenated C₁₋₆ alkoxy, wherein R' is selected from C₁₋₄ alkyl and C₁₋₆ alkoxycarbonyl, and R" is selected from H and C₁₋₄ alkyl. For said C₃₋₁₅ cycloalkyl, 6-14-membered aryl, 5-14-membered heteroaryl and 5-14-membered heterocyclic group in R²¹ and R²², further preferred substituent is -NR'R", wherein R' is selected from C₁₋₄ alkyl and C₁₋₆ alkoxycarbonyl, R" is selected from Hand C₁₋₄ alkyl.

Preferably, in the compound of Formula I, R²² is C₆₋₁₄ aryl or C₃₋₁₅ cycloalkyl C₁₋₆ alkyl that is optionally substituted by 1 or 2 substituents selected from the group consisting of halogen, C₁₋₆ alkyl, halogenated C₁₋₆ alkyl, -NH₂, -NR'R", C₁₋₆ alkoxy and halogenated C₁₋₆ alkoxy; more preferably C₆₋₁₄ aryl or C₃₋₁₅ cycloalkyl C₁₋₆ alkyl that is optionally substituted by one -NR'R", wherein R' is selected from C₁₋₄ alkyl and C₁₋₆ alkoxycarbonyl, R" is selected from Hand C₁₋₄ alkyl.

In some embodiments, the group -(CH₂)n¹-L-R²² is different from the group -(CH₂)n²-R²¹, wherein R²² is C₆₋₁₄ aryl or C₃₋₁₅ cycloalkyl C₁₋₆ alkyl that is optionally substituted by 1 or 2 substituents selected from the group consisting of halogen, C₁₋₆ alkyl, halogenated C₁₋₆ alkyl, -NH₂, -NR'R", C₁₋₆ alkoxy and halogenated C₁₋₆ alkoxy, wherein R' is selected from C₁₋₄ alkyl and C₁₋₆ alkoxycarbonyl, R" is selected from H and C₁₋₄ alkyl; R²¹ is H, halogen, C₁₋₆ alkyl, halogenated C₁₋₆ alkyl, C₂₋₆ alkenyl or C₂₋₆ alkynyl, and when R²¹ is H, n² is not 0. Preferably, R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl; the group -(CH₂)n²-R²¹ is C₁₋₆ alkyl or halogenated C₁₋₆ alkyl.

In one or more embodiments, R¹-R⁸ are hydrogen; L is ^{∗}-T₁-C(O)- or *- C(O)-T₁-, wherein T₁ is selected from NH or O; n¹ and n² are each integer of 1 to 6; the group -(CH₂)n²-R²¹ is C₁₋₆ alkyl; R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from Hand C₁₋₄ alkyl.

Preferably, in the compound of Formula I, the anion is selected from the group consisting of F⁻, Cl⁻, Br⁻, I⁻, NO₃⁻, SO₄²⁻, PO₄³⁻, HPO₄²⁻, H₂PO₄⁻, CO₃²⁻, HCO₃⁻, SO₃²⁻, HSO₃⁻, CH₃COO⁻ and CH₃SO₃⁻.

In one or more embodiments, the compound of Formula I according to the present disclosure has the structure represented by the following formula Ia, Ib or Ic: 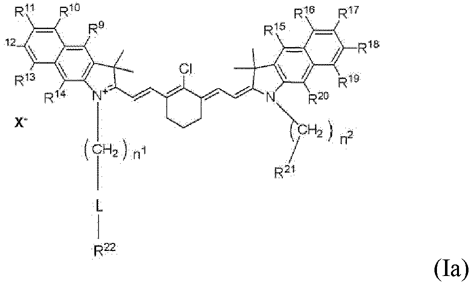 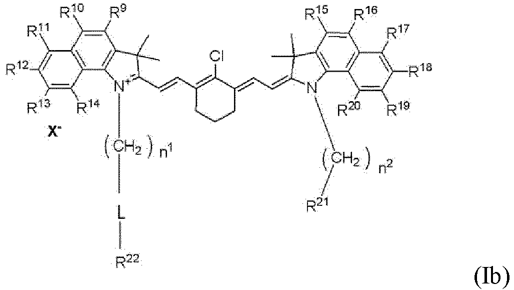 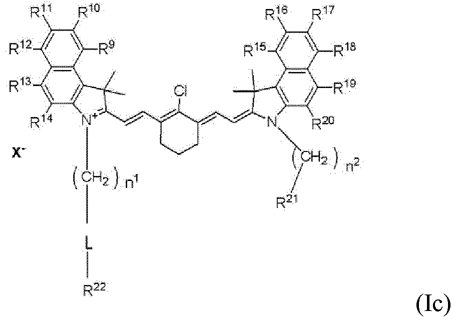
in each formula, n¹, n², L, R²¹ and R²² are as described in any of the foregoing embodiments;
R⁹-R²⁰ are each independently selected from the group consisting of H, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, C₃₋₁₀ cycloalkyl, C₆₋₁₄ aryl, C₅₋₁₄ heteroaryl and C₃₋₁₀ heterocyclic groups. Preferably, R⁹-R²⁰ are each independently H, halogen and C₁₋₆ alkyl, more preferably H.

Preferably, the compound of Formula I in the contrast agent is one or more selected from the group consisting of:

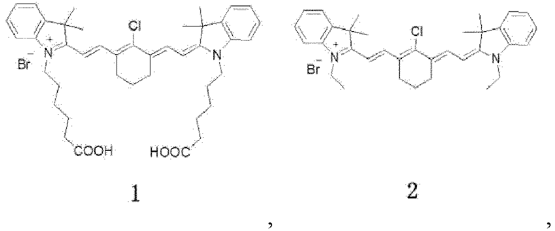

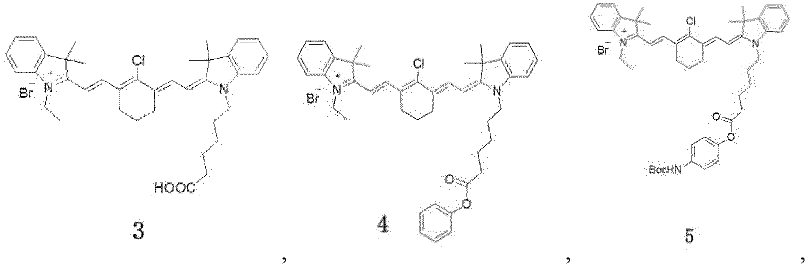

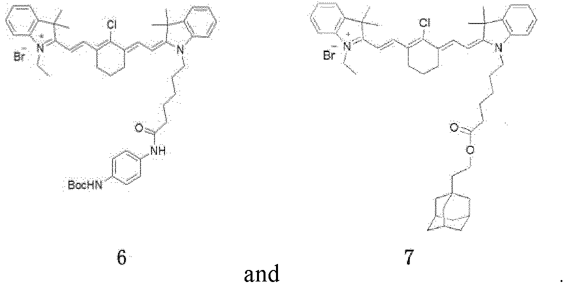

Preferably, the contrast agent of the present disclosure further comprises anionic surfactant. Suitable anionic surfactants include carboxylates, sulfonates, sulfate salts, phosphate ester salts and lipopeptide anionic surfactants. Preferable anionic surfactants include sulfonates and lipopeptide anionic surfactants, such as surfactin, sodium lauryl sulfonate and sodium lauryl benzene sulfonate, etc. In a particularly preferred embodiment, the anionic surfactant in the contrast agent of the present disclosure is surfactin (CAS No.: 24730-31-2). In the contrast agent of the present disclosure, the molar ratio of the compound of Formula I to the anionic surfactant in the contrast agent of the present disclosure is 1: (20-0.1), such as 1: (10-0.1) or 1: (5-0.2).

In the contrast agent of the present disclosure, the final concentration of the compound of Formula I in the contrast agent is in the range of 1 ∼ 100 µM, such as 5 ∼ 50 µM or 10 ∼ 30 µM.

Therefore, in a particularly preferred embodiment, the contrast agent of the present disclosure comprises the compound of Formula I and surfactin, wherein the compound of Formula I is as descrbied in any of the preceding embodiments. Preferably, in the contrast agent, R¹-R⁸ are all hydrogen, L is a bond, the group -(CH₂)n¹-L-R²² is the same as the group -(CH₂)n²-R²¹, for example, both of which are C₁₋₆ alkyl groups that are optionally substituted by 1 or 2 substituents selected from the group consisting of halogen, nitro, cyano, -SO₃, - COOH and -NH₂, preferably C₁₋₆ alkyl groups that are optionally substituted by -COOH. Preferably, in the compound of Formula I of the contrast agent, R¹-R⁸ are all hydrogen, L is ^{∗}-T₁-C(O)- or *- C(O)-T₁-, wherein T₁ is selected from NH or O; n¹ and n² are each integer of 1 to 6; R²² is C₆₋₁₄ aryl or C₃₋₁₅ cycloalkyl C₁₋₆ alkyl that is optionally substituted by 1 or 2 substituents selected from the group consisting of halogen, C₁₋₆ alkyl, halogenated C₁₋₆ alkyl, -NH₂, -NR'R", C₁₋₆ alkoxy and halogenated C₁₋₆ alkoxy, wherein R' is selected from C₁₋₄ alkyl and C₁₋₆ alkoxycarbonyl, R" is selected from H and C₁₋₄ alkyl; R²¹ is H, halogen, C₁₋₆ alkyl, halogenated C₁₋₆ alkyl, C₂₋₆ alkenyl or C₂₋₆ alkynyl, and when R²¹ is H, n² is not 0. Preferably, R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl; the group -(CH₂)n²-R²¹ is C₁₋₆ alkyl or halogenated C₁₋₆ alkyl.

Preferably, in the compound of Formula I in the contrast agent, R¹-R⁸ are hydrogen; L is ^{∗}-T₁-C(O)- or *- C(O)-T₁-, wherein T₁ is selected from NH or O; n¹ and n² are each integer of 1 to 6; the group -(CH₂)n²-R²¹ is C₁₋₆ alkyl; R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl. Preferably, in the contrast agent, the compound of Formula I is any one or more of Compounds 1-7, more preferably any one or more of Compounds 3-7, more preferably Compound 4 and/or Compound 6. Preferably, the molar ratio of the compound of Formula I to the surfactant in the contrast agent is 1: (10∼0.1), preferably 1: (5-0.2). Preferably, the final concentration of the compound of Formula I in the contrast agent is in the range of 5 ∼ 50 µM, preferably 10 ∼ 30 µM.

Preferably, the contrast agent of the present disclosure further comprises a solvent. The solvent should be able to dissolve the compound of Formula I of the present disclosure. Preferably, the solvent is water. In some embodiments, the contrast agent of the present disclosure is an aqueous solution of the compound of Formula I comprising the surfactant according to the present disclosure.

In a particularly preferred embodiment, the particle size of the contrast agent solution according to the present disclosure is 10-200nm, more preferably 30-150nm, more preferably 50-100nm.

The contrast agent of the present disclosure may be in the form of a solution, or may be in the form of a lyophilized powder.

In some embodiments, the present disclosure also provides a detection kit comprising the compound of Formula I according to any embodiment of the present disclosure. The compound of Formula I according to the present disclosure in the kit may be provided as an aqueous solution or as a solid powder. Preferably, the kit also contains anionic surfactant according to any embodiment herein. The ingredients in the kit can be individually packaged or supplied as a mixture. When individually packaged, the compound of Formula I of the present disclosure and an optional surfactant may be mixed in water to form a contrast agent before use. In some embodiments, the detection kit of the present disclosure comprises a contrast agent of the compound of Formula I according to the present disclosure. Preferably, the contrast agent containing the compound of Formula I of the present disclosure is the contrast agent as described in any embodiment of the present disclosure. The kit may also contain instructions instructing the technician to use the ingredients in the kit to make a contrast agent and/or instructing the technician to use the contrast agent for tumor tissue angiography.

### III. Compounds

In some embodiments, the present disclosure provides the compound of Formula I' as defined below: 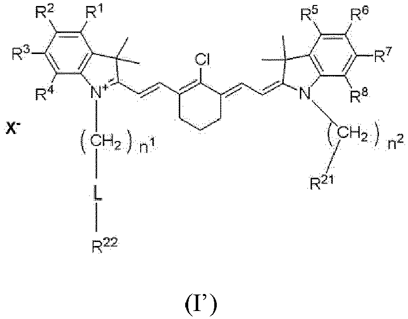 wherein,
X⁻ is anionic ion;
R¹-R⁸ are each independently H, hydroxyl, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, an optionally substituted C₃₋₁₀ cycloalkyl group, an optionally substituted 6-14-membered aryl group, an optionally substituted 5-14-membered heteroaryl group, and an optionally substituted 5-14-membered heterocyclic group; or R¹ and R², R² and R³ or R³ and R⁴ form an optionally substituted 3-8 membered carbon ring, 6-14 membered aromatic ring, 5-14 membered heteroaromatic ring or 5-14 membered hetero-ring with the carbon atoms to which they are attached, respectively, R⁵ and R⁶, R⁶ and R⁷ or R⁷ and R⁸ form an optionally substituted 3-8 membered carbon ring, 6-14 membered aromatic ring, 5-14 membered heteroaromatic ring or 5-14 membered hetero-ring with the carbon atoms to which they are attached, respectively;
R²¹ and R²² are each independently H, halogen, nitro, cyano, -SO₃, -COOH, -SO₃N(Rₐ)₃, C₁₋₆ alkyl, C₂₋₆ alkenyl, C₂₋₆ alkynyl, C₁₋₆ acyl, -NH₂, C₁₋₆ alkoxy, an optionally substituted C₃₋₁₅ cycloalkyl, an optionally substituted C₃₋₁₅ cycloalkyl C₁₋₆ alkyl, an optionally substituted 6-14-membered aryl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl; wherein each Rₐ is independently H and C₁₋₄ alkyl;
n¹ and n² are each independently selected from integers of 0-12, preferably integers of 0-6; and
L is a bond, or a linking group;
wherein the group -(CH₂)n¹-L-R²² is different from the group -(CH₂)n²-R²¹, and the steric hindrance of the group -(CH₂)n¹-L-R²² is greater than that of the group -(CH₂)n²-R²¹.

Preferably, in Formula I', R¹-R⁸ are each independently hydrogen, halogen or C₁-C₄ alkyl, preferably are all H.

Preferably, in Formula I', L is selected from the group consisting of ^{∗}-T₁-C(O)-, *-C(O)-T₁-, ^{∗}-S(O)₂-T₂- and ^{∗}-T₂-S(O)₂-, wherein T₁ is selected from the group consisting of NH, O and S, T₂ is selected from the group consisting of O, NH and S, * indicates the location in connection with -(CH₂)n¹-. More preferably, L is selected from ^{∗}-T₁-C(O)- and *- C(O)-T₁-, wherein T₁ is selected from NH and O.

Preferably, in R¹-R⁸ as well as R²¹ and R²² of Formula I', said an optionally substituted C₃₋₁₀ cycloalkyl, an optionally substituted 6-14-membered aryl, an optionally substituted 5-14-membered heteroaryl group, an optionally substituted 5-14-membered heterocyclic group, an optionally substituted C₃₋₁₅ cycloalkyl, an optionally substituted C₃₋₁₅ cycloalkyl C₁₋₆ alkyl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl are each an optionally substituted by 1-3 substitutents selected from the group consisting of halogen, C₁₋₆ alkyl, halogenated C₁₋₆ alkyl, hydroxyl, carboxyl, amino (including -NH₂ and -NR'R"), C₁₋₆ alkoxy and halogenated C₁₋₆ alkoxy, wherein R' is selected from C₁₋₄ alkyl and C₁₋₆ alkoxycarbonyl, and R" is selected from Hand C₁₋₄ alkyl.

Preferably, in Formula I', R²² is selected from the group consisting of an optionally substituted C₃₋₁₅ cycloalkyl, an optionally substituted C₃₋₁₅ cycloalkyl C₁₋₆ alkyl, an optionally substituted 6-14-membered aryl group, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl. Preferably, R²² is C₆₋₁₄ aryl or C₃₋₁₅ cycloalkyl C₁₋₆ alkyl that is optionally substituted by 1 or 2 substitutents selected from the group consisting of halogen, C₁₋₆ alkyl, halogenated C₁₋₆ alkyl, amino, C₁₋₆ alkoxy and halogenated C₁₋₆ alkoxy.

Preferably, in Formula I', R²¹ is H, halogen, nitro, cyano, -SO₃, -COOH, C₁₋₆ alkyl, C₂₋₆ alkenyl, C₂₋₆ alkynyl, C₁₋₆ acyl, -NH₂ or C₁₋₆ alkoxy, preferably H, C₁₋₆ alkyl, C₂₋₆ alkenyl, C₂₋₆ alkynyl, C₁₋₆ acyl, -NH₂ or C₁₋₆ alkoxy, more preferably H or C₁₋₆ alkyl.

Preferably, in Formula I', R²² is C₆₋₁₄ aryl or C₃₋₁₅ cycloalkyl C₁₋₆ alkyl that is optionally substituted by 1 or 2 substitutents selected from the group consisting of halogen, C₁₋₆ alkyl, halogenated C₁₋₆ alkyl, -NH₂, -NR'R", C₁₋₆ alkoxy and halogenated C₁₋₆ alkoxy, wherein R' is selected from C₁₋₄ alkyl and C₁₋₆ alkoxycarbonyl, R" is selected from H and C₁₋₄ alkyl; R²¹ is H, halogen, C₁₋₆ alkyl, halogenated C₁₋₆ alkyl, C₂₋₆ alkenyl or C₂₋₆ alkynyl, and when R²¹ is H, n² is not 0. Preferably, R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl; the group -(CH₂)n²-R²¹ is C₁₋₆ alkyl or halogenated C₁₋₆ alkyl.

Preferably, in Formula I', R¹-R⁸ are hydrogen; L is ^{∗}-T₁-C(O)- or *- C(O)-T₁-, wherein T₁ is selected from the group consisting of NH, O and S; n¹ and n² are each integer of 1 to 6; the group -(CH₂)n²-R²¹ is C₁₋₆ alkyl; R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from Hand C₁₋₄ alkyl.

Preferably, in Formula I', the anion is selected from the group consisting of F⁻, Cl⁻, Br⁻, I⁻, NO₃⁻, SO₄²⁻, PO₄³⁻, HPO₄²⁻, H₂PO₄⁻, CO₃²⁻, HCO₃⁻, SO₃²⁻, HSO₃⁻, CH₃COO⁻ and CH₃SO₃⁻.

In some embodiments, the compound of Formula I' according to the present disclosure has the structure represented by the following formula Ia', Ib' or Ic': 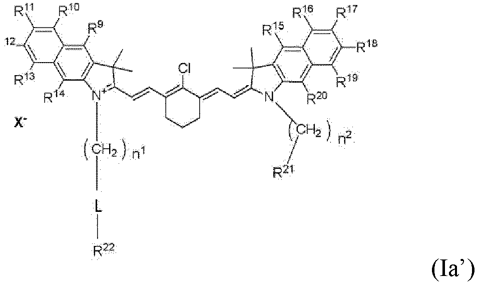 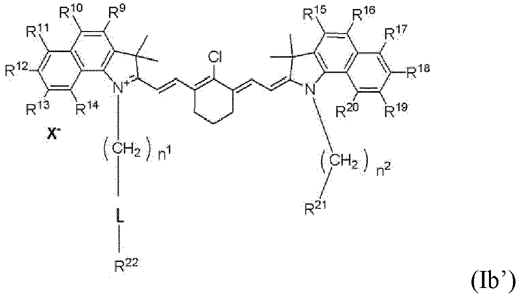 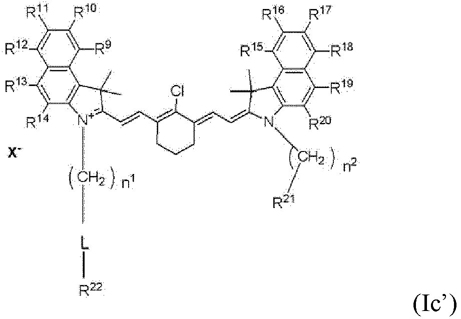
in each formula, n¹, n², L, R²¹ and R²² are as described in any of the foregoing embodiments of the compound of Formula I' according to the present disclosure;
R⁹-R²⁰ are each independently selected from the group consisting of H, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, C₃₋₁₀ cycloalkyl, C₆₋₁₄ aryl, C₅₋₁₄ heteroaryl and C₃₋₁₀ heterocyclic groups. Preferably, R⁹-R²⁰ are each independently H, halogen and C₁₋₆ alkyl, more preferably are all H.

In one or more preceding embodiments of the compound according to the present disclosure, the compound of Formula I' is selected from the group consisting of: 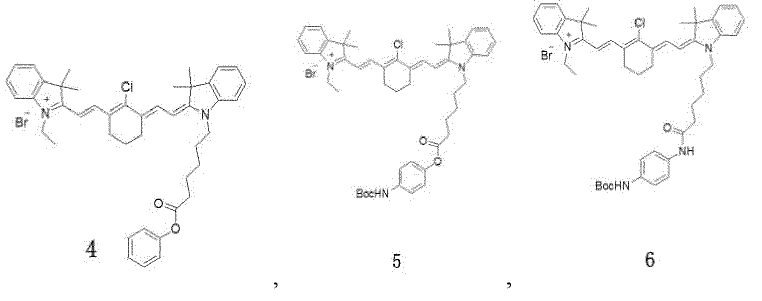 and

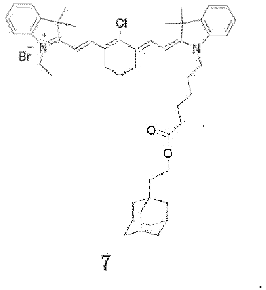

### IV. Preparation of the compound of Formula I

The present disclosure also provides a method for preparing the compound of Formula I', which comprises reacting the compound of Formula 1 with a compound Z-R²² to obtain the compound of Formula I': 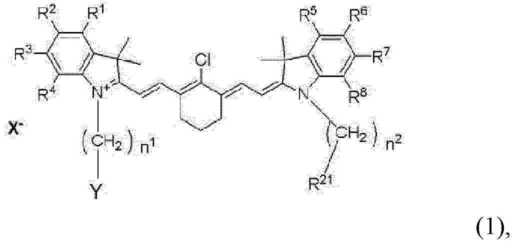 wherein X⁻, R¹-R⁸, n¹-n², R²¹ and R²² are as described in any of the preceding embodiments of Formula I'; Y and Z are selected such that L of the present disclosure, i.e., ^{∗}-T₁-C(O)-, *- C(O)-T₁-, ^{∗}-S(O)₂-T₂- and ^{∗}-T₂-S(O)₂- is obtained by reaction, wherein T₁ and T₂ are described in any of the preceding embodiments. For example, Y may be -NH₂, - COOH, -OH, -SH, -C(O)-X', -S(O)₂-X' or -S(O)₂OH, Z may correspondingly be a group that can react with Y, such as -NH₂, -COOH, -OH, -SH, -C(O)-X', -S(O)₂-X' or -S(O)₂OH, to form a group such as -C(O)-NH-, -NH-(CO)-, -C(O)-O-, -O-C(O)-, -S(O)₂-NH-, -NH-S(O)₂-, -S(O)₂-O-, -O-S(O)₂-, -S(O)₂-S-, -S-S(O)₂- and the like, wherein X' is halogen.

The reaction may be carried out at low temperatures (e.g. below 20°C) and under the protection of inert gases. The compound of Formula 1 and Z-R₂₂ may be dissolved in an organic solvent such as methylene chloride, tetrahydrofurand/or ethyl ether, and then slowly added with 1-equivalent of a salt of a condensing agent fromed with an acid (such as 1-(3-dimethylaminopropyl)-3-ethylcarbodiimide salt) and a catalyst (such as 4-dimethylaminopyridine) dissolved in the same organic solvent. The mixture is stirred at low speed. After the end of the reaction, dichloromethane is added, and the resultant is washed, dried, and purified when needed.

### V. Methods and uses

The contrast agent of the present disclosure may be used for tumor tissue angiography to distinguish tumor tissue from normal tissue. Unlike existing contrast agents, the contrast agent of the present disclosure can be used to treat tissues in vitro to develop tumor tissues. In some embodiments, the contrast agent of the present disclosure may also be used for in vivo administration.

Accordingly, the present disclosure provides a method for contrasting tumor tissues or cancer tissues in vitro, which comprises a step of contacting the tissues to be examined with a contrast agent or a compound of Formula I' of the present disclosure. Preferably, said contrast agent is the contrast agent described in any embodiment of the present disclosure. Contacting comprises immersing, smearing and/or spraying the tissue to be examined with the contrast agent of the present disclosure, so that the imaging of the tumor lesion is completed. Generally, after the tumor tissue is exposed to a solution containing the contrast agent or the compound of Formula I' of the present disclosure for a period of time, the tumor tissue or cancer tissue can be developed by imaging with a fluorescence imager.

The present disclosure also provides a use of a compound of Formula I and anionic surfactant in the preparation of a contrast agent for tumor tissues. The present disclosure also provides a use of the compound of Formula I' of the present disclosure in the preparation of a contrast agent for tumor tissues.

As confirmed by the Examples of the present disclosure, the targeted recognition of tumor tissues by other compounds of the present disclosure is achieved by organic anion transporting polypeptides (OATP). Therefore, any tumor or cancer tissue (especially solid tumors) expressing organic anion transporting polypeptides can be contrasted with the contrast agent or the compound of Formula I' and distinguished from the surrounding normal tissues. More particularly, the compounds of Formulas I and I' of the present disclosure, the contrast agents thereof, and the methods of the present disclosure may be used to contrast tumor tissues or cancer tissues selected from the group consisting of liver cancer, melanoma, neuroblastoma, breast cancer, ovarian cancer, lung cancer (small cell lung cancer and non-small cell lung cancer), Wilms tumor, cervical cancer, testicular cancer, soft tissue sarcoma, bladder cancer, primary brain cancer, gastric cancer, colon cancer, malignant pancreatic insulinoma, malignant carcinoid cancer, choriocarcinoma, mycosis fungoides, head and neck cancer, osteogenic sarcoma, pancreatic cancer, rhabdomyosarcoma, Kaposi sarcoma, thyroid cancer, esophageal cancer, kidney cancer, endometrial cancer, adrenal cortical adenocarcinoma, skin cancer and prostate cancer, etc.

In some embodiments, the compounds, the contrast agents and the methods of the present disclosure may be particularly useful for (1) early screening of cavity tumors, such as oral cavity cancer, esophageal cancer, gastric cancer, bowel cancer, colorectal cancer, bladder cancer, etc.; (2) intraoperative imaging of all known solid tumors, such as breast cancer, kidney cancer, brain cancer, etc., to check whether the surgical resection is complete; (3) photodynamic therapy of tumor lesions (mainly cavity tumors, such as bladder cancer, colorectal cancer, etc.).

The present disclosure has the following advantages:
(1) the product according to the present disclosure can perform high-contrast in vivo near-infrared imaging, and its imaging results are clear and distinguishable;
(2) the contrast agent according to the present disclosure does not need to be administered clinically by injection, and can be tested in vitro diagnostic tests;
(3) the production process of the products according to the present disclosure is simple and has significant advantages in production cost;
(4) The products according to the present disclosure are small molecule compounds with clear structure and high stability, and easy for storage and transportation.

The present disclosure will be described below referring to the specific examples. It should be understood that these examples are merely illustrative and are not intended to limit the present disclosure. The methods and reagents used in the examples, unless otherwise stated, are methods and reagents conventional in the art.

### Example 1: Preparation of the compound of Formula II

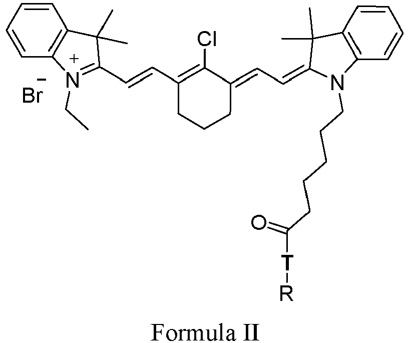

wherein T may be selected from O, NH or S; R is R₂₂ of the present disclosure.

The compound of Formula II may be prepared by the following steps:

At low temperature, under the protection of inert gas, Compound 3 and Compound 9 were dissolved in dichloromethane in an equivalent amount with stirring at low speed, and slowly added with 1 equivalent of 1-(3-dimethylaminopropyl)-3-ethylcarbodiimide hydrochloride and a catalytic amount of a solution of 4-dimethylaminopyridine in dichloromethane within 2 hours. The system was stirred at low speed for 24 hours. After the end of the reaction as monitored by silica gel thin layer chromatography, dichloromethane was added. The resultant was washed with saline, and dried with anhydrous sodium sulfate overnight. Silica gel column chromatography (methanol/dichloromethane) was used for purification, and the final purified product was a green powdery solid.

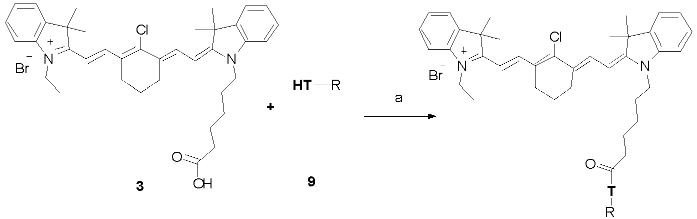

Compound 4 having the structure shown below was prepared according to the above method.

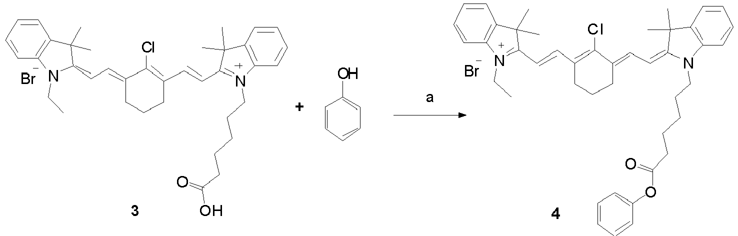

The chemical structure of Compound 4 was characterized as follows: ¹H NMR (500 MHz, CD₃Cl, *δ*): 8.37-8.31 (m, 2H), 7.54-7.53 (m, 1H), 7.41-7.35 (m, 7H), 7.25-7.12 (m, 4H), 7.03-7.01 (m, 1H), 6.32-6.31 (m, 1H), 6.30-6.29 (m, 1H), 4.32-4.26 (m, 4H), 2.76-2.73 (m, 4H), 2.63-2.60 (m, 2H), 1.96-1.83 (m, 8H), 1.72-1.71 (m, 12H), 1.47 (t, *J*=7.0 Hz, 3H).

¹³C NMR (125 MHz, CD₃Cl, *δ*): 172.1, 171.9, 150.6, 150.2, 144.5, 143.9, 129.4, 128.9, 127.6, 125.8, 125.3, 122.2, 121.5, 110.8, 101.5, 49.4, 49.2, 44.6, 39.9, 33.9, 31.9, 31.4, 30.2, 29.7, 29.4, 28.2, 28.0, 27.2, 26.6, 26.4, 24.5, 22.7, 20.7, 14.1, 12.5.

HRMS: *m*/*z* [M]⁺ calc for C₄₄H₅₀ClN₂O₂⁺: 673.3555; found:673.3550.

Compound 5 having the structure shown below was prepared according to the above method.

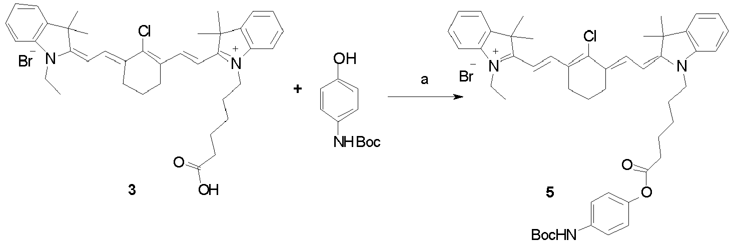

The chemical structure of Compound 5 was characterized as follows:
¹H NMR (500 MHz, CD₃Cl, *δ*): 8.38-8.29 (m, 2H), 7.43-7.37 (m, 7H), 7.25-7.21 (m, 2H), 7.15-7.14 (m, 1H), 6.95-6.93 (m, 2H), 6.38-6.35 (m, 1H), 6.26-6.23 (m, 1H), 4.36-4.32 (m, 2H), 4.24-4.21 (m, 2H), 2.76-2.70 (m, 4H), 2.60-2.57 (m, 2H), 1.93-1.85 (m, 8H), 1.72-1.71 (m, 12H), 1.51 (s, 9H), 1.47 (t, *J*=7.0 Hz, 3H).
¹³C NMR (125 MHz, CD₃Cl, *δ*): 172.4, 172.0, 171.6, 152.9, 150.2, 145.8, 144.8, 143.6, 136.4, 128.9, 128.0, 127.6, 125.6, 125.0, 122.2, 121.7, 110.8, 110.5, 101.9, 101.1,49.5,49.1, 44.4, 40.0, 33.8, 31.2, 29.7, 28.3, 28.1, 28.0, 27.1, 26.6, 26.3, 24.5, 22.7, 20.7, 14.4, 12.5.
HRMS: *m*/*z* [M]⁺ calc for C₄₉H₅₉ClN₃O₄⁺: 788.4189; found:788.4185.

Compound 6 having the structure shown below was prepared according to the above method.

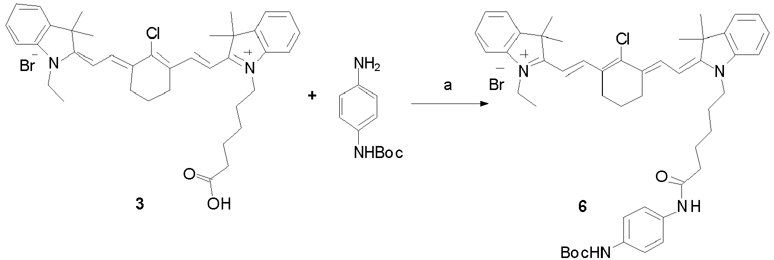

The chemical structure of Compound 6 was characterized as follows:
¹H NMR (500 MHz, CD₃Cl, *δ*): 10.69 (s, 1H), 8.42-8.39 (m, 1H), 8.28-8.24 (m, 1H), 7.91-7.89 (m, 2H), 7.43-7.35 (m, 5H), 7.31-7.29 (m, 1H), 7.24-7.17 (m, 3H), 7.05-7.03 (m, 1H), 6.43-6.40 (m, 1H), 5.99-5.96 (m, 1H), 4.28-4.25 (m, 2H), 4.07-4.02 (m, 2H), 2.75-2.73 (m, 2H), 2.71-2.68 (m, 2H), 2.62-2.59 (m, 2H), 1.90-1.82 (m, 8H), 1.71-1.70 (m, 12H), 1.50 (s, 9H), 1.43 (t, *J*=7.0 Hz, 3H).
¹³C NMR (125 MHz, CD₃Cl, *δ*): 174.4, 172.7, 169.7, 150.7, 146.5, 142.5, 142.1, 141.7, 141.1, 140.7, 129.2, 128.7, 128.3, 127.1, 126.2, 126.1, 124.5, 122.3, 122.2, 120.6, 111.9, 109.6, 103.3, 98.8, 49.5, 48.8, 45.0, 38.9, 36.7, 31.2, 28.4, 28.1, 28.0, 26.9, 26.6, 26.4, 26.0, 25.2, 20.6, 12.4, 12.0.
HRMS: *m*/*z* [M]⁺ calc for C₄₉H₆₀ClN₄O₃⁺: 787.4348; found:787.4346.

Compound 7 having the structure shown below was prepared according to the above method.

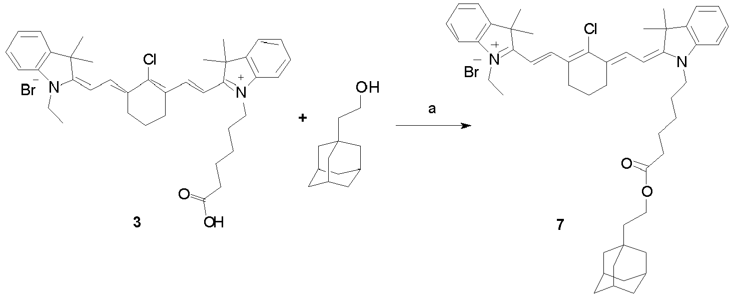

The chemical structure of Compound 7 was characterized as follows:
¹H NMR (500 MHz, CD₃Cl, *δ*): 10.69 (s, 1H), 8.42-8.39 (m, 1H), 8.28-8.24 (m, 1H), 7.91-7.89 (m, 2H), 7.43-7.35 (m, 5H), 7.31-7.29 (m, 1H), 7.24-7.17 (m, 3H), 7.05-7.03 (m, 1H), 6.43-6.40 (m, 1H), 5.99-5.96 (m, 1H), 4.28-4.25 (m, 2H), 4.07-4.02 (m, 2H), 2.75-2.73 (m, 2H), 2.71-2.68 (m, 2H), 2.62-2.59 (m, 2H), 1.90-1.82 (m, 8H), 1.71-1.70 (m, 12H), 1.50 (s, 9H), 1.43 (t, *J*=7.0 Hz, 3H).
¹³C NMR (125 MHz, CD₃Cl, *δ*): 174.4, 172.7, 169.7, 150.7, 146.5, 142.5, 142.1, 141.7, 141.1, 140.7, 129.2, 128.7, 128.3, 127.1, 126.2, 126.1, 124.5, 122.3, 122.2, 120.6, 111.9, 109.6, 103.3, 98.8, 49.5, 48.8, 45.0, 38.9, 36.7, 31.2, 28.4, 28.1, 28.0, 26.9, 26.6, 26.4, 26.0, 25.2, 20.6, 12.4, 12.0.
HRMS: *m*/*z* [M]⁺ calc for C₄₉H₆₀ClN₄O₃⁺: 787.4348; found:787.4346.

Compounds 1, 2, 3 and 8 used in the examples have the following structures, respectively:

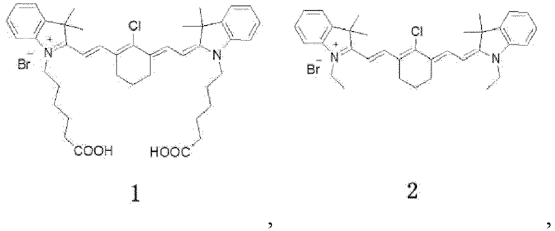

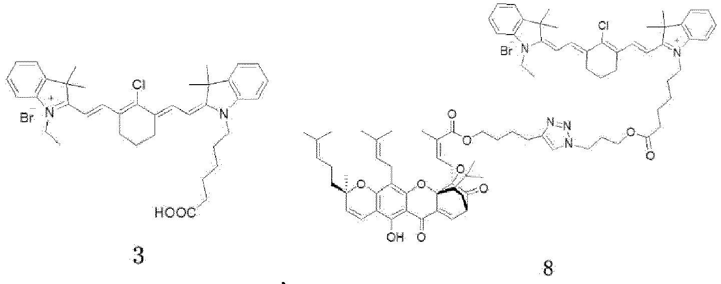

Compound 1 is reported in a reference, Near-infrared fluorescence imaging of cancer mediated by tumor hypoxia and HIF1alpha/OATPs signaling axis. Biomaterials 2014, 35, 8175-8185;

Compound 2 is reported in a reference, A self-assembled "albumin-conjugate" nanoprobe for near infrared optical imaging of subcutaneous and metastatic tumors. ACS Appl. Bio Mater. 2020, 3, 327-334;

Compounds 3 and 8 are reported in a reference, A novel "mosaic-type" nanoparticle for selective drug release targeting hypoxic cancer cells. Nanoscale 2019, 11, 2211-2222.

Example 2: Fluorescence localization imaging of Compounds 1-3, 4, 5, and 7 in human renal cancer cell line 786o.

786o cells (3×10⁵/well) were seeded in a 6-well plate in advance and placed in a cell culture incubator for 48 h. After the culture medium was blotted, a culture medium comprising the corresponding compound (5µM) and Mito-tracker (0.2nM) was added to the experimental well, incubated for 30 min, cleaned three times with PBS solution, and imaged using a fluorescence microscope equipped with an infrared camera and a continuous LED light source. The near-infrared photos were taken under Ex=745 nm, Em=820 nm; the Mito-tracker photos were taken under Ex=488 nm and Em=520 nm.

The experimental results for Compounds 1-3, 4, 5, and 7 are shown in Figs. 1-6, respectively.

The results showed that Compounds 1-3, 4, 5 and 7 could be taken up by tumor cells, and their intracellular distribution could basically be colocalized with the Mito-tracker distribution, which could label the mitochondria of cells, so this experiment proved at the cellular level that such compounds could enter tumor cells and be enriched in mitochondria.

Example 3: Fluorescence localization imaging of Compounds 4 and 5 in human renal cancer cell line A498.

The same method as Example 2 was employed except that the human renal cancer cell line A498 was used to detect the localization of Compounds 4, 5 within the cell.

The experimental results for Compounds 4 and 5 are shown in Figs. 7 and 8. The results showed that Compounds 4, 5 in human renal cancer cell line A498 could also be uptaken by tumor cells and enriched in mitochondria.

Example 4: Fluorescence quenching effect after mixing Compounds 1-5, 7 and Surfactin as anionic surfactant in different ratios.

Surfactin in different concentrations was added to the aqueous solution of the corresponding compound (final concentration of 20 µM), and the molar ratio of the compound to be tested to surfactin was 1:5, 1:1 and 1:0.2. A methanol solution of the corresponding compound (final concentration of 20 µM) was used as a positive control. The test solution was shaken for 5 min and added to a black 96-well plate with 50ul of test solution per experimental well. Then a fluorescence imager was used for imaging. The photos were taken under Ex=745 nm, Em=820 nm. The ROI (Region of Interest) value for each well was quantitatively calculated.

The results are shown in Fig. 9. The results showed that: (1) compared with the methanol solution, obvious fluorescence quenching of the compound in the aqueous solution can occur under the action of surfactin, and the fluorescence quenching effect is still obvious even when the molar ratio of the compound to surfactin decreases to 1:0.2; (2) the fluorescence quenching effect of Compounds 4, 5 and 7 is better than that of Compounds 1, 2 and 3, and the fluorescence quenching effect of Compound 1 is the worst.

Example 5: Fluorescence emission spectroscopy test after mixing Compounds 1-8 with Surfactin as anionic surfactant in different ratios.

Surfactin in different concentrations was added to the aqueous solution of the corresponding compound (final concentration of 20µM), and the molar ratio of the compound to be tested to surfactin was 1:5, 1:1, 1:0.2. A methanol solution of the corresponding compound (final concentration of 20 µM) was used as a positive control. The test solution was shaken for 5 minutes and its fluorescence emission spectra (Ex=730 nm, Em=760-960 nm) were detected using a fluorescence spectrophotometer.

The experimental results of Compounds 1-8 are shown in Figs.10-17, respectively.

The results showed that compared with the methanol solution, obvious fluorescence quenching of Compound 1 in the aqueous solution could occur under the action of surfactin (the molar ratio of Compound 1 to surfactin was 1:5); when the amount of surfactin was reduced, the fluorescence quenching effect was significantly reduced. Compared with the methanol solution, obvious fluorescence quenching of Compound 2 in the aqueous solution could occur under the action of surfactin (the molar ratio of Compound 2 to surfactant was 1:5); when the amount of surfactin was reduced, the fluorescence quenching effect was significantly reduced. Compared with the methanol solution, obvious fluorescence quenching of Compound 3 in the aqueous solution could occur under the action of surfactin (the molar ratio of Compound 3 to surfactant was 1:5); when the amount of surfactin was reduced, the fluorescence quenching effect was significantly reduced. Compared with the methanol solution, obvious fluorescence quenching of Compound 4 in the aqueous solution could occur under the action of surfactin; when the amount of surfactin was reduced, the fluorescence quenching effect was not significantly reduced. Compared with the methanol solution, obvious fluorescence quenching of Compound 5 in the aqueous solution could occur under the action of surfactin; when the amount of surfactin was reduced, the fluorescence quenching effect was not significantly reduced. Compared with the methanol solution, obvious fluorescence quenching of Compound 6 in the aqueous solution could occur under the action of surfactin; when the amount of surfactin was reduced, the fluorescence quenching effect was not significantly reduced. Compared with the methanol solution, obvious fluorescence quenching of Compound 7 in the aqueous solution could occur under the action of surfactin; when the amount of surfactin was reduced, the fluorescence quenching effect was not significantly reduced. Compared with the methanol solution, obvious fluorescence quenching of Compound 8 in the aqueous solution could occur under the action of surfactin; when the amount of surfactin was reduced, the fluorescence quenching effect was not significantly reduced.

Example 6: Fluorescence emission spectroscopy test after mixing Compound 4 with various surfactants.

Different surfactants were added to the aqueous solution of Compound 4 (final concentration of 20µM), and the molar ratio of the compound to be tested to surfactin was 1:1. In addition, a methanol solution of the compound (final concentration of 20 µM) was used as a positive control. The test solution was shaken for 5 minutes and its fluorescence emission spectra (Ex=730 nm, Em=760-960 nm) were detected using a fluorescence spectrophotometer.

The experimental results are shown in Fig. 18. In Fig. 18, each curve represents from top to bottom: dodecyltrimethylammonium chloride (5), polyethylene oxide-polypropylene oxide-polyethylene oxide triblock copolymer (P123, 4), polyoxyethylene castor oil (Kolliphor, 3), human serum albumin (2), tetrabutylammonium iodide (6), sodium dodecyl sulfonate (7), sodium dodecylbenzenesulfonate (8) and surfactin (1), of which 7, 8 and 1 substantially coincide.

The results showed that human serum albumin, nonionic surfactants (Kolliphor, P123) and cationic surfactants (dodecyltrimethylammonium chloride, tetrabutylammonium iodide) mixed with Compound 4 in the aqueous solution could reduce their fluorescence signal intensity, but there was still fluorescence signal in the solution. A fluorescence quenching effect occurred after anionic surfactants (surfactin, sodium dodecyl sulfonate, sodium dodecyl benzene sulfonate) were mixed with Compound 4 in the aqueous solution, and there was substantially no fluorescence signal in the solution.

Example 7: Microscopic morphological test of Compounds 4, 5, 7 mixed with Surfactin in a molar ratio of 1:5 in an aqueous solution.

Surfactin (final concentration of 100 µM) was added to the aqueous solution of the corresponding compound (final concentration of 20 µM), and the molar ratio of the compound to be tested to surfactin was 1:5. The test solution was shaken for 5 minutes and its micromorphology was detected using a Transmission Electron Microscope (TEM).

The experimental results are shown in Fig. 19. The results showed that Compounds 4, 5 and 7 could form particles with a particle size of 50-100 nm after mixing with Surfactin in a molar ratio of 1:5 in the aqueous solution.

Example 8: Near-infrared imaging of Compounds 1-8 in a mouse subcutaneous xenograft renal clear cell carcinoma tumor model.

Female nude mice around 5 weeks of age were ordered and subjected to subcutaneous renal clear cell cancer cell 786o inoculation after one week of barrier environmental adaptation at a specific position above the right lower limb (3×10⁶/pce, suspended in 200 µl PBS solution). When the tumor volume reached 50-150mm³ (about 12-22 days), nude mice were randomly grouped with 3 mice in each experimental group. Mice were dissected, and tumor tissues were taken.

Compounds 1-8 were added to the aqueous solution of surfactin. The final concentration of surfactin was 100 µM, and the final concentration of each compound was 20 µM (the molar ratio of the compound to surfactant was 1:5).

The tumor tissue was immersed in the above liquid. A fluorescence imager was used for imaging after a specified time.

The experimental results of Compounds 1-8 are shown in Figs. 20-27, respectively. As shown in Figs. 20-27, the results of ROI numerical quantitative analysis of tumor tissue showed that: (1) There was no obvious positive correlation between the uptake of Compounds 1, 2, 3 and 8 by tumor tissues and the immersion time, so for the experimental group of Compounds 1, 2, 3 and 8, the reason for near-infrared imaging of tumor tissues was more the non-specific adsorption of the compounds by tissues than tumor target recognition. (2) The uptake of Compounds 5 and 7 by tumor tissues increases with the immersion time, which has a certain positive correlation, and the reason for near-infrared imaging of tumor tissues is the targeted recognition of tumor tissue by the compounds. However, the tumor tissue has a low uptake of the compounds. (3) The uptake of Compounds 4 and 6 by tumor tissues increases with the immersion time, which has an obvious positive correlation, and the reason for near-infrared imaging of tumor tissues is the targeted recognition of tumor tissues by the compounds. Tumor tissue has a high uptake of the compounds. Therefore, Compounds 4 and 6 have optimal imaging of tumor tissue.

### Example 9: Near-infrared imaging of Compounds 4 and 6 in a mouse subcutaneous xenograft breast cancer tumor model

Using the method described in Example 8, a mouse subcutaneous xenograft breast cancer tumor model was constructed, and then the mouse model was used to detect the near-infrared imaging of Compounds 4,6 (in aqueous solution, the molar ratio of the compound to surfactin is 1:5) in the tumor tissue.

The experimental results for Compounds 4 and 6 are shown in Figs. 28 and 29, respectively. As shown in Figs. 28 and 29, the results of ROI numerical quantitative analysis of tumor tissues showed that the uptake of Compounds 4 and 6 by tumor tissues also increased with the immersion time in mouse subcutaneous xenograft breast cancer tumor models, which had an obvious positive correlation, and the uptake of the compounds by tumor tissues was higher.

Example 10: Near-infrared imaging of Compound 4 under excessive competition in a mouse subcutaneous xenograft renal clear cell carcinoma tumor model.

Using the method described in Example 8, a mouse subcutaneous xenograft renal clear cell carcinoma tumor model was constructed, and then the mouse model was used to detect the near-infrared imaging of Compound 4 (in aqueous solution, the molar ratio of the compound to surfactin was 1:5 with a final concentration of 1 mM of sodium bromosulfophthalein (50 equivalent) added) in the tumor tissue.

The experimental results are shown in Fig. 30. Sodium bromosulfophthalein is a reported inhibitor of organic anion transporting polypeptides (OATPs). The experimental results showed that the use of excessive sodium bromosulfophthalein could effectively reduce the uptake of Compound 4 by tumor tissues. Thus, it can be deduced that target recognition of tumor tissues by Compound 4 and other compounds of the present disclosure is achieved by the organic anion transporting polypeptides.

Example 11: Near-infrared imaging of mixtures of Compounds 1-8 and surfactin (the molar ratio of the compound to surfactin was 1:5) as anionic surfactant in a mouse primary bowel cancer tumor model.

Establishment of the mouse primary bowel cancer tumor model of APCmin/+ mouse model: parent C57BL/6J-APC min/+ mice and C57BL/6J wt mice were caged and propagated. The genotype of the offspring was detected and the offspring C57BL/6J-APC min/+ mice (diseased mice) were screened. The offspring C57BL/6J-APC min/+ mice were killed at week 20. Mice were dissected and diseased colon tissue was taken. Colon was cut longitudinally for subsequent tests.

Compounds 1-8 were added to the aqueous solution of surfactin. The final concentration of surfactin was 100 µM, and the final concentration of the compound was 20 µM (the molar ratio of the compound to surfactin was 1:5).

The diseased colon tissue of mice was immersed in the above fluid. A fluorescence imager was used for imaging after 12 min.

The experimental results of Compounds 1-8 are shown in Figs. 31-38, respectively.

The experimental results showed that, after short-term immersion contact, the mixtures of Compounds 1, 2 and 3 and surfactin as anionic surfactant (the molar ratio of the compound to surfactin was 1:5) had a recognition effect on tumor lesions in mouse primary bowel cancer tumor models. The mixtures of Compounds 4, 5, 6 and surfactant as anionic surfactant (the molar ratio of the compound to surfactant was 1:5) have a high specific recognition on tumor lesions in mouse primary bowel cancer tumor models. The tumor lesion tissues had significantly higher fluorescence intensity than that of normal intestinal tissues, with excellent signal-to-noise ratio and clearly distinguishable imaging results. Compound 4 and Compound 6 were best imaged. The mixture of Compound 7 and surfactin as anionic surfactant (the molar ratio of the compound to surfactin was 1:5) had a recognition effect on tumor lesion tissues in mouse primary bowel cancer tumor models. The mixture of Compound 8 and surfactin as anionic surfactant (the molar ratio of the compound to surfactin was 1:5) had substantially no recognition effect on tumor lesion tissues in mouse primary bowel cancer tumor models. Both of tumor lesion tissues and normal intestinal tissues had relatively weak fluorescence signals. The signal-to-noise ratio of the imaging results was very poor with no obvious imaging effect.

## Claims

1. A contrast agent comprising a compound of Formula I and anionic surfactant: 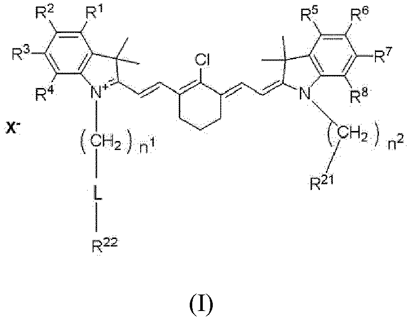 wherein,
X⁻ is anionic ion;
R¹-R⁸ are each independently H, hydroxyl, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, an optionally substituted C₃₋₁₀ cycloalkyl group, an optionally substituted 6-14 membered aryl group, an optionally substituted 5-14 membered heteroaryl group, and an optionally substituted 5-14 membered heterocyclic group; or R¹ and R², R² and R³ or R³ and R⁴ form an optionally substituted 3-8 membered carbon ring, 6-14 membered aromatic ring, 5-14 membered heteroaromatic ring or 5-14 membered hetero-ring with the carbon atoms to which they are attached, respectively, R⁵ and R⁶, R⁶ and R⁷ or R⁷ and R⁸ form an optionally substituted 3-8 membered carbon ring, 6-14 membered aromatic ring, 5-14 membered heteroaromatic ring or 5-14 membered hetero-ring with the carbon atoms to which they are attached, respectively;
R²¹ and R²² are each independently H, halogen, nitro, cyano, -SO₃, -COOH, -SO₃N(Rₐ)₃, C₁₋₆ alkyl, C₂₋₆ alkenyl, C₂₋₆ alkynyl, C₁₋₆ acyl, -NH₂, C₁₋₆ alkoxy, an optionally substituted C₃₋₁₅ cycloalkyl, an optionally substituted C₃₋₁₅ cycloalkyl C₁₋₆ alkyl, an optionally substituted 6-14-membered aryl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl; wherein each Rₐ is independently H and C₁₋₄ alkyl;
n¹ and n² are each independently selected from integers of 0-12, preferably integers of 0-6; and
L is a bond, or a linking group.

2. The contrast agent according to claim 1, wherein
the group -(CH₂)n¹-L-R²² is the same as the group -(CH₂)n²-R²¹, both of which are C₁₋₆ alkyl groups that are optionally substituted by 1 or 2 substituents selected from the group consisting of halogen, nitro, cyano, -SO₃, -COOH and -NH₂, preferably C₁₋₆ alkyl groups that are optionally substituted by -COOH; and/or
R¹-R⁸ are all hydrogen.

3. The contrast agent according to claim 1, wherein
the group -(CH₂)n¹-L-R²² is different from the group -(CH₂)n²-R²¹, and the steric hindrance of the group -(CH₂)n¹-L-R²² is greater than that of the group -(CH₂)n²-R²¹; preferably, R²² is selected from the group consisting of an optionally substituted C₃₋₁₅ cycloalkyl, an optionally substituted C₃₋₁₅ cycloalkyl C₁₋₆ alkyl, an optionally substituted 6-14-membered aryl group, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl, R²¹ is H, halogen, nitro, cyano, -SO₃, -COOH, C₁₋₆ alkyl, C₂₋₆ alkenyl, C₂₋₆ alkynyl, C₁₋₆ acyl, -NH₂ or C₁₋₆ alkoxy, preferably H, C₁₋₆ alkyl, C₂₋₆ alkenyl, C₂₋₆ alkynyl, C₁₋₆ acyl, -NH₂ or C₁₋₆ alkoxy, more preferably H or C₁₋₆ alkyl; and/or
R¹-R⁸ are all hydrogen; and/or
L is selected from the group consisting of ^{∗}-T₁-C(O)-, *- C(O)-T₁-, ^{∗}-S(O)₂-T₂- and *-T₂-S(O)₂-, wherein T₁ is selected from the group consisting of NH, O and S, T₂ is selected from the group consisting of O, NH and S, * indicates the location in connection with - (CH₂)n¹-; preferably, L is selected from ^{∗}-T₁-C(O)- or *- C(O)-T₁-, wherein T₁ is preferably NH or O.

4. The contrast agent according to claim 1, wherein
the group -(CH₂)n¹-L-R²² is different from the group -(CH₂)n²-R²¹, the group -(CH₂)n¹-L-R²² is C₁₋₆ alkyl that is optionally substituted by 1 or 2 substituents selected from the group consisting of halogen, nitro, cyano, -SO₃, -COOH and -NH₂, preferably C₁₋₆ alkyl that is optionally substituted by -COOH; the group -(CH₂)n²-R²¹ is C₁₋₆ alkyl that is optionally substituted by 1 or 2 substituents selected from the group consisting of halogen, nitro, cyano, -SO₃, -COOH and -NH₂, preferably C₁₋₆ alkyl that is optionally substituted by -COOH, and the carbon chain length of the group -(CH₂)n²-R²¹ is shorter than the carbon chain length of the group -(CH₂)n¹-L-R²²; preferably, the group -(CH₂)n¹-L-R²² is C₁₋₆ alkyl that is optionally substituted by -COOH, the group -(CH₂)n²-R²¹ is C₁₋₆ alkyl that is optionally substituted by -COOH, and the carbon chain length of the group -(CH₂)n²-R²¹ is shorter than the carbon chain length of the group -(CH₂)n¹-L-R²²; more preferably, the group -(CH₂)n¹-L-R²² is C₁₋₆ alkyl that is substituted by -COOH, the group -(CH₂)n²-R²¹ is an unsubstituted C₁₋₆ alkyl, and the carbon chain length of the group -(CH₂)n²-R²¹ is shorter than the carbon chain length of the group -(CH₂)n¹-L-R²²; and
R¹-R⁸ are all hydrogen.

5. The contrast agent according to claim 1, wherein
R¹-R⁸ are hydrogen;
L is ^{∗}-T₁-C(O)- or *- C(O)-T₁-, wherein T₁ is selected from NH or O;
n¹ and n² are each intergers of 1-6;
the group -(CH₂)n²-R²¹ is C₁₋₆ alkyl; and
R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl.

6. The contrast agent according to claim 1, wherein the compound of Formula I has structures represented by the following Formula Ia, Ib or Ic: 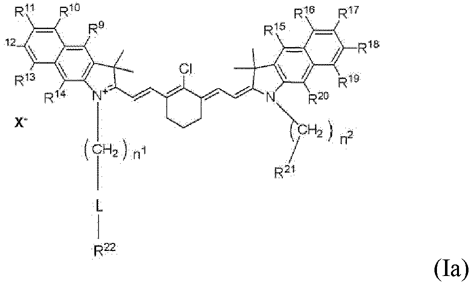 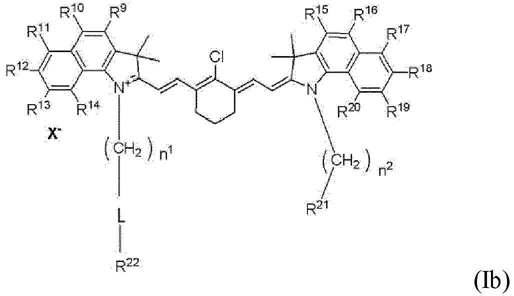 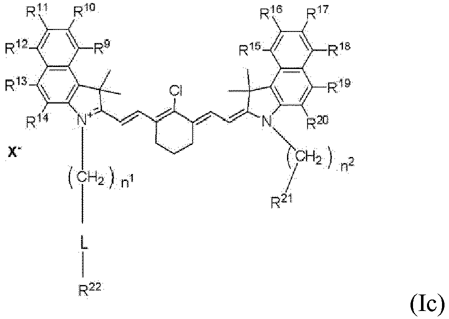
in each formula, n¹, n², L, R²¹ and R²² are as defined in any one of claims 1-6;
R⁹-R²⁰ are each independently selected from the group consisting of H, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, C₃₋₁₀ cycloalkyl, C₆₋₁₄ aryl, C₅₋₁₄ heteroaryl and C₃₋₁₀ heterocyclic groups; preferably, R⁹-R²⁰ are each independently H, halogen and C₁₋₆ alkyl, more preferably H.

7. The contrast agent according to claim 1, wherein the anion ion is selected from the group consisting of F⁻, Cl⁻, Br⁻, I⁻, NO₃⁻, SO₄²⁻, PO₄³⁻, HPO₄²⁻, H₂PO₄⁻, CO₃²⁻, HCO₃⁻, SO₃²⁻, HSO₃⁻, CH₃COO⁻ and CH₃SO₃⁻.

8. The contrast agent according to claim 1, wherein the compound of Formula I is one or more compounds selected from the following compounds:

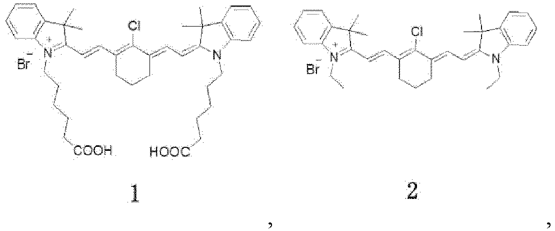

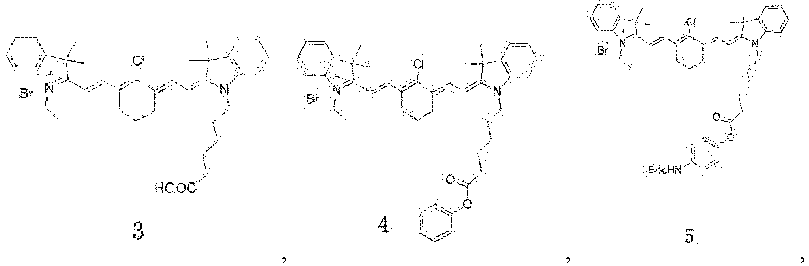

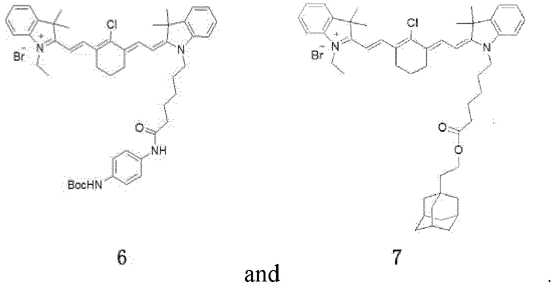

9. The contrast agent according to any one of claims 1-8, wherein
the anionic surfactant is selected from carboxylates, sulfonates, sulfate salts, phosphate ester salts and lipopeptide anionic surfactants; preferably sulfonates and lipopeptide anionic surfactants; more preferably surfactin, sodium lauryl sulfonate and/or sodium lauryl benzene sulfonate; and/or
the molar ratio of the compound of Formula I to the anionic surfactant is 1: (20~0.1), such as 1: (10-0.1) or 1: (5-0.2); and/or
the final concentration of the compound of Formula I in the contrast agent is in the range of 1 ∼ 100 µM, preferably 5 ~ 50 µM or 10 ∼ 30 µM; and/or
the particle size of the contrast agent solution is 10-200nm, preferably 30-150nm, more preferably 50-100nm; and/or
the contrast agent is in a form of a solution or a lyophilized powder.

10. A detection kit comprising the compound of Formula I according to any one of claims 1-8 and anionic surfactant; preferably, the anionic surfactant is as defined in claim 9;
preferably, the detection kit comprises the contrast agent as defined in any one of claims 1-9.

11. A compound of Formula I' defined as follows: 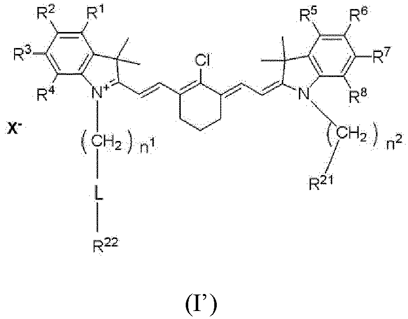 wherein,
X⁻ is anionic ion;
R¹-R⁸ are each independently H, hydroxyl, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, an optionally substituted C₃₋₁₀ cycloalkyl group, an optionally substituted 6-14 membered aryl group, an optionally substituted 5-14 membered heteroaryl group, and an optionally substituted 5-14 membered heterocyclic group; or R¹ and R², R² and R³ or R³ and R⁴ form an optionally substituted 3-8 membered carbon ring, 6-14 membered aromatic ring, 5-14 membered heteroaromatic ring or 5-14 membered hetero-ring with the carbon atoms to which they are attached, respectively, R⁵ and R⁶, R⁶ and R⁷ or R⁷ and R⁸ form an optionally substituted 3-8 membered carbon ring, 6-14 membered aromatic ring, 5-14 membered heteroaromatic ring or 5-14 membered hetero-ring with the carbon atoms to which they are attached, respectively;
R²¹ and R²² are each independently H, halogen, nitro, cyano, -SO₃, -COOH, -SO₃N(Rₐ)₃, C₁₋₆ alkyl, C₂₋₆ alkenyl, C₂₋₆ alkynyl, C₁₋₆ acyl, -NH₂, C₁₋₆ alkoxy, an optionally substituted C₃₋₁₅ cycloalkyl, an optionally substituted C₃₋₁₅ cycloalkyl C₁₋₆ alkyl, an optionally substituted 6-14-membered aryl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl; wherein each Rₐ is independently H and C₁₋₄ alkyl;
n¹ and n² are each independently selected from integers of 0-12, preferably integers of 0-6; and
L is a bond, or a linking group;
wherein the group -(CH₂)n¹-L-R²² is different from the group -(CH₂)n²-R²¹, and steric hindrance of the group -(CH₂)n¹-L-R²² is greater than that of the group -(CH₂)n²-R²¹.

12. The compound according to claim 11, wherein
R¹-R⁸ are all hydrogen; and/or
L is selected from the group consisting of ^{∗}-T₁-C(O)-, *- C(O)-T₁-, ^{∗}-S(O)₂-T₂- and *-T₂-S(O)₂-, wherein T₁ is selected from the group consisting of NH, O and S, T₂ is selected from the group consisting of O, NH and S, ^{∗} indicates the location in connection with - (CH₂)n¹-; preferably, L is selected from ^{∗}-T₁-C(O)- and ^{∗}-C(O)-T₁-, wherein T₁ is selected from NH and O; and/or
R²² is selected from the group consisting of an optionally substituted C₃₋₁₅ cycloalkyl, an optionally substituted C₃₋₁₅ cycloalkyl C₁₋₆ alkyl, an optionally substituted 6-14-membered aryl, an optionally substituted 6-14-membered arylalkyl group, an optionally substituted 5-14-membered heteroaryl group, an optionally substituted 5-14-membered heteroaryl C₁₋₆ alkyl, an optionally substituted 5-14-membered heterocyclic group and an optionally substituted 5-14-membered heterocyclic group C₁₋₆ alkyl; preferably, R²² is C₆₋₁₄ aryl or C₃₋₁₅ cycloalkyl C₁₋₆ alkyl that is optionally substituted by one or two substitutents selected from the group consisting of halogen, C₁₋₆ alkyl, halogenated C₁₋₆ alkyl, amino, C₁₋₆ alkoxy and halogenated C₁₋₆ alkoxy; most preferably, R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl; and/or
R²¹ is H, halogen, nitro, cyano, -SO₃, -COOH, C₁₋₆ alkyl, C₂₋₆ alkenyl, C₂₋₆ alkynyl, C₁₋₆ acyl, -NH₂ or C₁₋₆ alkoxy, preferably H, C₁₋₆ alkyl, C₂₋₆ alkenyl, C₂₋₆ alkynyl, C₁₋₆ acyl,-NH₂ or C₁₋₆ alkoxy, more preferably H or C₁₋₆ alkyl.

13. The compound according to claim 11, wherein
R¹-R⁸ are hydrogen;
L is ^{∗}-T₁-C(O)- or *- C(O)-T₁-, wherein T₁ is selected from the group consisting of NH, O and S; n¹ and n² are each intergers of 1-6;
the group -(CH₂)n²-R²¹ is C₁₋₆ alkyl;
R²² is C₆₋₁₄ aryl or C₃₋₁₅ cycloalkyl C₁₋₆ alkyl that is optionally substituted by -NR'R", wherein R' is selected from C₁₋₄ alkyl and C₁₋₆ alkoxycarbonyl, R" is selected from H and C₁₋₄ alkyl.

14. The compound of claim 11, wherein the anionic is selected from the group consisting of F⁻, Cl⁻, Br⁻, I⁻, NO₃⁻, SO₄²⁻, PO₄³⁻, HPO₄²⁻, H₂PO₄⁻, CO₃²⁻, HCO₃⁻, SO₃²⁻, HSO₃⁻, CH₃COO⁻ and CH₃SO₃⁻.

15. The compound of claim 11, wherein the compound of Formula I' has the structure represented by the following formula Ia', Ib' or Ic': 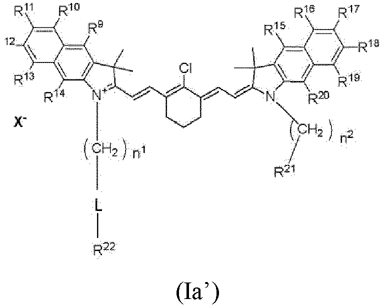 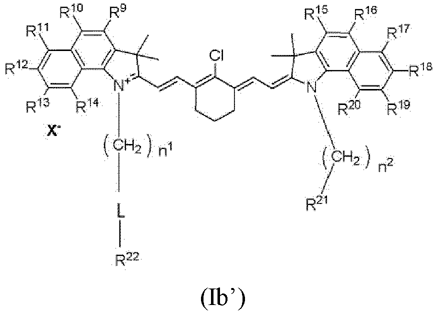 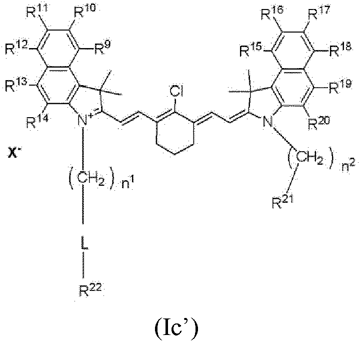
in each formula, X⁻, n¹, n², L, R²¹ and R²² are as defined in any of claims 11-14;
R⁹-R²⁰ are each independently selected from the group consisting of H, halogen, nitro, cyano, -SO₃, -COOH, C₁₋₆ alkyl, C₂₋₆ alkenyl, C₂₋₆ alkynyl, C₁₋₆ acyl, -NH₂, C₁₋₆ alkoxy, C₃₋₁₀ cycloalkyl, C₆₋₁₄ aryl, C₅₋₁₄ heteroaryl and C₃₋₁₀ heterocyclic groups, preferably, R⁹-R²⁰ are each independently H, halogen and C₁₋₆ alkyl, more preferably are all H.

16. The compound of claim 11, wherein the compound of Formula I' is selected from the group consisting of: 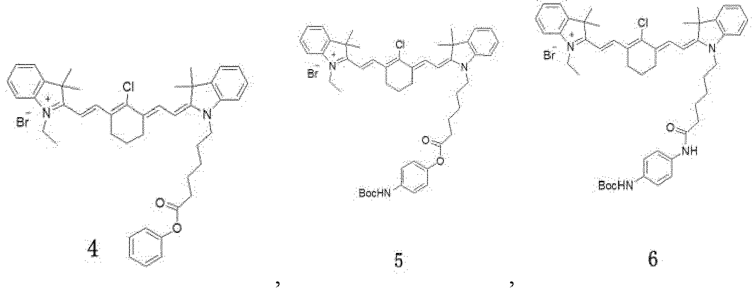 and

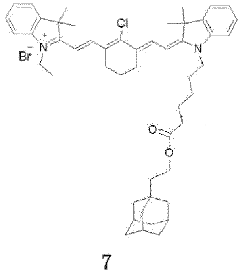

17. A contrast agent comprising the compound of Formula I' according to any one of claims 11-16 and an optional anionic surfactant; preferably, the the anionic surfactant is as defined in claim 9.

18. A detection kit comprising the compound of Formula I' according to any one of claims 11-16 and an optional anionic surfactant; preferably, the anionic surfactant is as defined in claim 9; preferably, the detection kit comprises the contrast agent as defined in claim 17.

19. Use of the compound of Formula I' according to any one of claims 11-16 and an optional anionic surfactant in the manufacture of a tumor contrast agent; use of the compound of Formula I according to any one of claims 1-8 and anionic surfactant in the manufacture of a tumor contrast agent; preferably, the anionic surfactant is as defined in claim 9.

20. A contrast agent or a detection kit comprising the contrast agent, wherein the contrast agent comprises Compound 4 and/or Compound 6 and anionic surfactant: 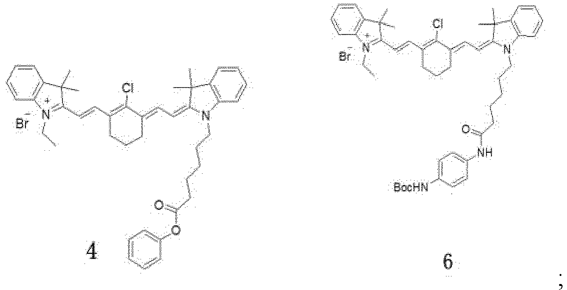
wherein the anionic surfactant is one or more selected from the group consisting of surfactin, sodium lauryl sulfonate and sodium lauryl benzene sulfonate, wherein in the contrast agent, the molar ratio of Compound 4, Compound 6, or Compound 4 and Compound 6 to the anionic surfactant is in the range of 1: (20∼0.1), preferably 1: (10-0. 1) or 1: (5-0.2);
preferably, the contrast agent is in the form of a solution or a lyophilized powder.
